# EUROPEAN PATENT SPECIFICATION

(11) **EP 3 727 429 B1**
(45) Date of publication and mention of the grant of the patent: **07.05.2025**
(21) Application number: 18825698.6
(22) Date of filing: 20.12.2018
(51) Int. Cl.: A61K 38/48, A61P 21/00

(54) **TREATMENT OF AUTONOMIC DISORDERS WITH BOTULINUM TOXIN**
BEHANDLUNG VON AUTONOMEN STÖRUNGEN MIT DER BOTULINISCHE TOXINE
TRAITEMENT DES TROUBLES AUTONOMIQUES PAR LA TOXINE BOTULINIQUE

(30) Priority: 20.12.2017 EP 17306840
(43) Date of publication of application: 28.10.2020
(73) Proprietor: Ipsen Biopharm Limited, Wrexham LL13 9UF (GB)
(72) Inventor: KRUPP, Johannes, Wrexham LL13 9UF (GB); MAIGNEL-LUDOL, Jacquie, Wrexham LL13 9UF (GB); PIGNOL, Bernadette, Wrexham LL13 9UF (GB)
(74) Representative: Hobson, David James
(86) International application number: PCT/EP2018/086261
(87) International publication number: WO 2019/122166

(56) References cited:
- WO-A1-2016/154534
- WO-A1-2016/156113
- WO-A1-2017/191315
- MARC A. BENSON ET AL: "Unique Ganglioside Recognition Strategies for Clostridial Neurotoxins", JOURNAL OF BIOLOGICAL CHEMISTRY, vol. 286, no. 39, 30 September 2011 (2011-09-30), US, pages 34015 - 34022, XP055485187, ISSN: 0021-9258, DOI: 10.1074/jbc.M111.272054

## Description

The present invention relates to the treatment of autonomic disorders with neurotoxins, as claimed.

Bacteria in the genus Clostridia produce highly potent and specific protein toxins, whicn can poison neurons and other cells to which they are delivered. Examples of such clostridial toxins include the neurotoxins produced by *C*. *tetani* (TeNT) and by *C*. *botulinum* (BoNT) serotypes A-G, as well as those produced by *C*. *baratii* and *C. butyricum.*

Clostridial neurotoxins cause muscle paralysis by inhibiting cholinergic transmission in the peripheral nervous system, in particular at the neuromuscular junction, and can thus be lethal. In nature, clostridial neurotoxins are synthesised as a single-chain polypeptide that is modified post-translationally by a proteolytic cleavage event to form two polypeptide chains joined together by a disulphide bond. Cleavage occurs at a specific cleavage site, often referred to as the activation site, which is located between the cysteine residues that provide the inter-chain disulphide bond. It is this di-chain form that is the active form of the toxin. The two chains are termed the heavy chain (H-chain), which has a molecular mass of approximately 100 kDa, and the light chain (L-chain), which has a molecular mass of approximately 50 kDa. The H-chain comprises an N-terminal translocation component (H_{N} domain) and a C-terminal targeting component (H_{C} domain). The cleavage site is located between the L-chain and the H_{N} domain.

The mode of action of clostridial neurotoxins relies on five distinct steps: (1) binding of the H_{C} domain to the cell membrane of its target neuron, followed by (2) internalisation of the bound toxin into the cell via an endosome, (3) translocation of the L-chain by the H_{N} domain across the endosomal membrane and into the cytosol, (4) proteolytic cleavage of intracellular transport proteins known as SNARE proteins by the L-chain which provides a non-cytotoxic protease function, and (5) inhibition of cellular secretion from the target cell.

In this cascade of events, SNARE proteins (Soluble N-ethylmaleimide-Sensitive Factor Attachment protein REceptor) are integral to intracellular vesicle fusion, and thus to secretion of molecules via vesicle transport from a cell. Examples of SNARE proteins present in neurons include, among others, SNAP-25, VAMP, or Syntaxin. The non-cytotoxic protease function of the L-chain is, on the other hand, a zinc-dependent endopeptidase activity, which exhibits a high substrate specificity for SNARE proteins. Accordingly, once delivered to a neuronal target cell, the non-cytotoxic protease of clostridial neurotoxins, by cleaving its substrate SNARE protein, inhibits neurotransmitter release, which consequently leads to neuroparalysis.

Thanks to their unique properties, Clostridial neurotoxins, such as botulinum toxin, have been successfully employed in a wide range of therapeutic applications, in particular for motor and autonomic disorders, to restore for example the activity of hyperactive nerve endings to normal levels. At least seven antigenically distinct BoNTs serotypes have been described so far, namely BoNT/A, BoNT/B, BoNT/C, BoNT/D, BoNT/E, BoNT/F, BoNT/G (Rossetto, O. et al., "Botulinum neurotoxins: genetic, structural and mechanistic insights." Nature Reviews Microbiology 12.8 (2014): 535-549). Despite this diversity, BoNT/A remains the serotype of choice in therapy, with three commonly available commercial preparations (Botox^{®}, Dysport^{®} and Xeomin^{®}), while only one BoNT/B product is available on the market (Neurobloc^{®}/Myobloc^{®}). To this day, these BoNT/A and BoNT/B products, which are toxins purified from clostridial strains, are the only two BoNT serotypes that are currently approved by regulatory agencies for use in humans for applications ranging, among others, from spasticity, bladder dysfunction, or hyperhidrosis (for BoNT/A) (see for example https://www.medicines.org.uk/emc/medicine/112,https://www.medicines.org.uk/emc/medicine /870, https://www.medicines.org.uk/emc/medicine/2162)
to cervical dystonia (for BoNT/B) (see for example, https://www.medicines.org.uk/emc/medicine/20568).

However, a current limitation observed in their therapeutic uses is the generation of neutralizing antibodies in patients, which renders future treatment ineffective. Though few have reported antigenicity issues with Neurobloc^{®}/MyoBloc^{®}, such antibody responses have been so far mainly reported with BoNT/A products which remain the botulinum toxin of choice. These immunological responses have been identified as being directly correlated to toxin doses (Lange, O., et al., Neutralizing antibodies and secondary therapy failure after treatment with botulinum toxin type A: much ado about nothing? Clin Neuropharmacol, 2009, 32, 213-218). Adverse effects may also result from diffusion of toxins to other regions of the body and the possibility of toxin diffusion is directly related to injected doses.

Because both the generation of neutralizing antibodies and toxin diffusion are directly related to injected doses, there is a need in the art to treat patients with products, such as alternative BoNT serotypes, which are capable of achieving the same level of therapeutic effect with lower doses.

A challenge in this regard is the difference in *modus operandi* of the various BoNT serotypes, which seem to translate into a difference in therapeutic efficacy for the same disorder.

Indeed, in order to exert clinical activity, the botulinum toxin has to gain entry into the neuronal end terminal of its target cell. To do so, BoNTs target and enter neurons by binding to their specific receptors through their receptor binding domains (BoNT-H_{C}), which are welldefined in the literature (Schiavo, G., Matteoli, M. & Montecucco, C. Neurotoxins affecting neuroexocytosis, Physiol Rev, 2000, 80, 717-766). Receptor binding generally dictates the efficacy and specificity of BoNTs to recognize neurons. BoNT/B, D-C, and G share two homologous synaptic vesicle proteins synaptotagmin I and II (Syt I/II) as their receptors, while BoNT/A, E, D, and F use another synaptic vesicle protein SV2. In this regard, it should be noted that the binding affinity of BoNT serotypes for their protein receptors can also vary from species to species: for example, BoNT/B, /D-C and /G display a much higher affinity for Sytll than Sytl at motor nerve terminals in rodents, while, in humans, these have a greater affinity for Sytl than Sytll - this difference being due to a unique amino acid change between the rodent and human Sytll sequences. As a result of this amino acid change, human Sytll is significantly less efficient in mediating entry of BoNT/B, /D-C and /G, as compared to mouse Sytll. This also means that, in humans, the high affinity receptor for BoNT/B, /D-C and /G appears to be restricted to the minor receptor Sytl, at least in motoneurons. All of these findings may provide an explanation for the clinical observations that a much higher dose of BoNT/B than BoNT/A (which binds a different receptor) appears to be needed to achieve the same levels of therapeutic effects in cervical dystonia patients (Brashear A. et al., Safety and efficacy of NeuroBloc (botulinum toxin type B) in type A-resistant cervical dystonia, 1999, Neurology 53, 1431-1438 ; Pappert, E.J. & Germanson, T. Botulinum toxin type B vs. type A in toxin-naive patients with cervical dystonia: Randomized, double-blind, noninferiority trial, 2008 Mov Disord 23, 510-517). In particular, for use at neuromuscular junction, a conversion ratio between BOTOX^{®} and MyoBloc^{™} of 1:40 has been suggested (Dressler et al., Botulinum toxin type B for treatment of axillar hyperhidrosis., 2002, J. Neurol. 249,1729-1732; Comella et al., 2005).

In addition to protein receptors, all BoNTs serotypes require lipid co-receptor gangliosides which are abundant on neuronal surfaces and can differ between serotypes, and cleave different SNARE protein substrates, which may further impact their therapeutic efficacy depending on the cell type targeted. Indeed, the L-chain proteases of BoNT/B, BoNT/D, BoNT/F and BoNT/G cleave VAMP, while the L-chain proteases of BoNT/A and BoNT/E cleave SNAP25 and the L-chain protease of BoNT/C cleaves both SNAP25 and syntaxin. Even though the initial therapeutic uses of botulinum neurotoxins relied upon the inhibition of acetylcholine release at the neuromuscular junction of skeletal muscles to treat neuromuscular conditions such as dystonias and spasticity, it has later been found that these neurotoxins were also effective on glands and smooth muscles by inhibiting acetylcholine release at autonomic nerve terminals, and could thus be used to treat a variety of autonomic disorders such as hyperhidrosis, sialorrhea or overactive bladder.

The recommended dose of Botox^{®} (also known as Onabotulinumtoxin A) for treating hyperhidrosis is 50 units per axilla (https://www.medicines.orq.uk/emc/medicine/112), that is a dose of about 0.365 ng of the 150 kD BoNT/A toxin. Neurobloc^{®}/Myobloc^{®} (also known as Rimabotulinumtoxin B) is not approved for treating hyperhidrosis; however, doses ranging from 2000 units to 4000 units have been disclosed in the literature, i.e. doses ranging from about 4-40 ng of the 150 kD BoNT/A toxin. Such high dosages are however not without side effects, both at the regional and systemic level, such as dryness of mouth, dysphagia, heartburn, etc (Dressler D. and Eleopra R., Clinical use of non-A botulinum toxins: botulinum toxin type B., Neurotoxicity research 9.2-3 (2006): 121-125; Tintner R. et al., Autonomic function after botulinum toxin type A or B: a double-blind, randomized trial., Neurology 65.5 (2005): 765-767; Birklein F. et al. Botulinum toxin type B blocks sudomotor function effectively: a 6 month follow up., Journal of investigative dermatology 121.6 (2003): 1312-1316). Nevertheless, it has not been investigated whether therapeutic efficacy could be reached for autonomic disorders with lower doses of toxin.

WO 2017/191315 describes chimeric neurotoxins with enhanced properties and their use in therapy.

WO 2016/154534 describes a botulinum neurotoxin (BoNT) polypeptide with a modified receptor binding domain (Hc) having one or more amino acid mutations that modify the binding of the BoNT to the receptor.

WO 2016/156113 describes recombinant clostridial neurotoxins which have been modified to improve their activation.

Benson et al. (J Biol Chem. 2011 Sep 30;286(39):34015-22) describes unique ganglioside recognition strategies for clostridial neurotoxins.

The present invention aims at providing improved treatment of autonomic disorders, that circumvent the clinical issues currently observed in patients, such as production of neutralizing antibodies against the toxin, as well as regional and/or systemic side effects.

The present invention provides a clostridial neurotoxin for use in the treatment of an autonomic disorder in a human patient, wherein the clostridial neurotoxin comprises an H_{CC} domain from a BoNT/B, BoNT/F, BoNT/D, BoNT/D-C, or BoNT/G, and wherein a dose ranging from 0.00025 ng to 3 ng of said clostridial neurotoxin is administered to the patient.

The dose of the clostridial neurotoxin to be administered to the patient may be equivalent to or lower than the dose of BoNT/A treating said autonomic disorder.

The dose of said clostridial neurotoxin to be administered to the patient may be 1.1 times to 100 times, preferably 1.3 times to 90 times, lower than the dose of BoNT/A treating said autonomic disorder.

The invention provides a clostridial neurotoxin for use in the treatment of an autonomic disorder in a human patient, wherein said clostridial neurotoxin comprises a H_{CC} domain from a BoNT/B, BoNT/D, BoNT/D-C, BoNT/F or BoNT/G, and wherein a dose ranging from 0.00025 ng to 3 ng of said clostridial neurotoxin is to be administered to said patient.

The present invention is firstly based on the unexpected finding by the Inventors that BoNT/B is at least as potent as BoNT/A on human smooth muscle tissue.

This finding is unexpected since, as mentioned above, much higher doses of BoNT/B (Neurobloc^{®}/Myobloc^{®}) are required for treating skeletal muscle disorders as compared to BoNT/A (e.g. Botox^{®}). It is likely based on this clinical observation that only higher doses of BoNT/B (Neurobloc^{®}/Myobloc^{®}) than BoNT/A (eg Botox^{®}) have been so far tested and reported in the literature for the treatment of e.g. hyperhidrosis which involves smooth muscles.

It is hypothesized that this difference in BoNT/B potency in these two tissues is due to the fact that smooth and skeletal muscle disorders are actually not dependent upon the same nervous routes. Smooth muscle contractions are indeed driven by the autonomic nervous system whereas skeletal muscle contractions are driven by the somatic nervous system. Cervical dystonia and many other disorders treated with botulinum neurotoxins such as spasticity are caused by excessive contractions of skeletal muscle, whereas other disorders such as overactive bladder (OAB) or neurogenic detrusor overactivity (NDO) are caused by excessive contractions of smooth muscles.

The somatic nervous system (SoNS or voluntary nervous system) is the part of the peripheral nervous system associated with skeletal muscle voluntary control of body movements. The SoNS consists of afferent nerves and efferent nerves. SoNS afferent nerves are responsible for relaying sensation from the body to the central nervous system (CNS) and SoNS efferent nerves are responsible for sending out commands from the CNS to the body, stimulating muscle contraction; they include all the non-sensory neurons connected with skeletal muscles and skin.

The autonomic nervous system (ANS) is another component of the peripheral nervous system and also consists of afferent nerves and efferent nerves. The ANS is made up of two branches: the sympathetic nervous system (SNS) and the parasympathetic nervous system (PNS). The SNS controls the more active responses such as increasing heart rate and blood pressure. In addition to smooth muscles, the ANS also controls secretions such as sweat, saliva and tears.

Smooth muscles are found in the urinary system (e.g. bladder, ureters), in the digestive system (e.g. stomach walls, gastrointestinal tract, intestines, sphincter of Oddi, anal sphincter, trachea, bile duct), in the reproductive system (e.g. prostate, uterus), in the respiratory tract, in the vascular system (e.g. blood vessel walls, aorta, arteries, arterioles, veins) and in the iris of the eye. Smooth muscle contractions are responsible for a wide number of body functions such as releasing urine from the bladder, moving food through the digestive tract, regulating air flow in lungs, regulating blood pressure in arteries and veins, and shrinking the size of the pupil.

The synaptic nerve endings of cholinergic neurons supplying eccrine sweat glands, salivary glands, and lacrimal glands can be targeted with botulinum neurotoxins. Neurogenic disorders associated with sudomotor or secretomotor hyperactivity include hyperhidrosis, in particular focal hyperhidrosis of the palms, axillae, or feet, gustatory sweating (Frey syndrome), increased tearing during eating (crocodile tears syndrome), hypersalivation (sialorrhea, drooling), etc.

Without wishing to be bound by theory, it is hypothesized that the difference in potency observed between BoNT/A and BoNT/B on the somatic system versus the autonomic system is related to the fact that BoNT/A and BoNT/B differ with respect to their protein receptor (SV2 and Syt I/II respectively), and/or to their target SNARE (SNAP25 and VAMP respectively).

Based on this assumption, it has further been hypothesized that other BoNT serotypes which also use Syt I/II as a protein receptor and/or cleave VAMP, such as BoNT/D, BoNT/D-C, BoNT/F or BoNT/G, will display a similar potency as BoNT/B, if not a better potency, towards smooth muscles / the autonomic system, at least in humans.

The dose of the clostridial neurotoxin to be administered to the patient may be equivalent to or lower than the dose of BoNT/A treating said autonomic disorder.

In one embodiment, the autonomic disorder to be treated by the clostridial neurotoxin of the invention is selected from the group consisting of smooth muscle disorders, hypersecretory disorders, respiratory disorders, inflammatory disorders with an autonomic component, neuroendocrine disorders and other autonomic disorders directly associated with a central neurological disorder.

Examples of such autonomic disorders include, without limitation:
- smooth muscle disorders, that are notably caused by excessive, abnormal and/or prolonged muscle contractions (spastic disorders), including e.g.:
   o urinary disorders, such as neurogenic detrusor overactivity (NDO), overactive bladder (OAB), in particular idiopathic OAB (iOAB), bladders spasms, detrusor-sphincter dyssynergia (DSD), urinary incontinence, urinary retention, nocturia, urge incontinence, urinary frequency; gastrointestinal disorders, such as sphincter of Oddi dysfunction, esophageal spasms, intestinal spasms, achalasia, gastroparesis, spastic colitis, anal fissures, constipation, occasional diarrhoea, dysphagia, oropharyngeal dysphagia , swallowing disorders;
   ∘ vascular and cardiovascular disorders such as Raynaud's disease, anastomotic thrombosis, atrial fibrillation after cardiac surgery, orthostatic hypotension, blood pressure disorder;
   ∘ prostate disorders such as benign prostate hyperplasia (BPH), prostatitis, prostodynia, prostatic enlargement; and
   o sexual disorders: erectile dysfunction, priapism, ejaculatory failure, vaginism;
- hypersecretory disorders, such as hyperhidrosis (axillary, palmar, plantar hyperhidrosis, diffuse sweating, Frey's syndrome, etc.), hypersalivation (drooling, sialorrhea), gustatory sweating, excessive lacrimation, crocodile tears syndrome, excessive mucus secretion, bromhidrosis, stomach acid secretion, acne;
- respiratory disorders which have a hypersecretory and/or muscular component, such as rhinorrhea, chronic rhinitis, asthma, chronic obstructive pulmonary disease (COPD), bronchial hyperreactivity, stridor, involuntary inspiratory gasps, apnoeic episodes;
- inflammatory disorders which have an autonomic component, such as otitis media, itch, pruritis, inflammatory bowel syndrome;
- neuroendocrine disorders: diabetes, hyperinsulinism, hyperglucagonism pancreatic disorders, thyroid disorders, hypocalcemia, hyperthyroidism, metabolic disorders, excessive lipolysis; and
- other autonomic disorders directly associated with central neurological disorders such as parkinsonian and cerebellar/pyramidal features.

In a preferred embodiment, the autonomic disorder to be treated according to the invention is a smooth muscle disorder selected from the group consisting of urinary disorders, gastrointestinal disorders, vascular and cardiovascular disorders, prostate disorders and sexual disorders.

The term "clostridial neurotoxin" as used herein means any polypeptide that enters a neuron and inhibits neurotransmitter release. This process encompasses the binding of the neurotoxin to a low or high affinity receptor, the internalisation of the neurotoxin, the translocation of the endopeptidase portion of the neurotoxin into the cytoplasm and the enzymatic modification of the neurotoxin substrate. More specifically, the term "neurotoxin" encompasses any polypeptide produced by *Clostridium* bacteria (clostridial neurotoxins) that enters a neuron and inhibits neurotransmitter release, and such polypeptides produced by recombinant technologies or chemical techniques. It is this di-chain form that is the active form of the toxin. The two chains are termed the heavy chain (H-chain), which has a molecular mass of approximately 100 kDa, and the light chain (L-chain), which has a molecular mass of approximately 50 kDa. Preferably, the clostridial neurotoxin is a botulinum neurotoxin (BoNT).

BoNT serotypes A to G can be distinguished based on inactivation by specific neutralising anti-sera, with such classification by serotype correlating with percentage sequence identity at the amino acid level. BoNT proteins of a given serotype are further divided into different subtypes on the basis of amino acid percentage sequence identity.

An example of a BoNT/A neurotoxin amino acid sequence is provided as SEQ ID NO: 1 (UniProt accession number A5HZZ9). An example of a BoNT/B neurotoxin amino acid sequence is provided as SEQ ID NO: 2 (UniProt accession number B1INP5). An example of a BoNT/C neurotoxin amino acid sequence is provided as SEQ ID NO: 3 (UniProt accession number P18640). An example of a BoNT/D neurotoxin amino acid sequence is provided as SEQ ID NO: 4 (UniProt accession number P19321). An example of a BoNT/E neurotoxin amino acid sequence is provided as SEQ ID NO: 5 (accession number WP_003372387). An example of a BoNT/F neurotoxin amino acid sequence is provided as SEQ ID NO: 6 (UniProt accession number Q57236) or as SEQ ID NO: 11 (UniProt/UniParc accession number UP10001DE3DAC). An example of a BoNT/G neurotoxin amino acid sequence is provided as SEQ ID NO: 7 (accession number WP_039635782). An example of a BoNT/D-C neurotoxin amino acid sequence is provided as SEQ ID NO: 8 (accession number BAM65681).

The term "H_{C} domain" as used herein refers to a functionally distinct region of the neurotoxin heavy chain with a molecular weight of approximately 50 kDa that enables the binding of the neurotoxin to a receptor located on the surface of the target cell. The H_{C} domain consists of two structurally distinct subdomains, the "H_{CN} subdomain" (N-terminal part of the H_{C} domain) and the "H_{CC} subdomain" (C-terminal part of the H_{C} domain, also named H_{CC} domain), each of which having a molecular weight of approximately 25 kDa. A H_{CC} domain is capable of binding to a clostridial neurotoxin protein receptor.

The term "LH_{N} domain" as used herein refers to a neurotoxin region that is distinct from the H_{C} domain, and which consists of an endopeptidase domain ("L" or "light chain") and of a domain responsible for translocation of the endopeptidase into the cytoplasm (H_{N} domain of the heavy chain). An endopeptidase domain ("L" or "light chain") is capable of cleaving a SNARE protein.

Exemplary L, H_{N}, H_{CN} and H_{CC} domains are shown in table 1.

**Table 1 - Exemplary L, H_{N}, H_{CN} and H_{CC} domains**

| **BoNT** | **Accession Number** | **SEQ ID NO:** | **L** | **H_{N}** | **H_{CN}** | **H_{CC}** |
|---|---|---|---|---|---|---|
| BoNT/A1 | A5HZZ9 | 1 | 1-448 | 449-872 | 873-1094 | 1095-1296 |
| BoNT/B1 | B1INP5 | 2 | 1-441 | 442-859 | 860-1081 | 1082-1291 |
| BoNT/C1 | P18640 | 3 | 1-449 | 450-867 | 868-1095 | 1096-1291 |
| BoNT/D | P19321 | 4 | 1-442 | 443-863 | 864-1082 | 1083-1276 |
| BoNT/E1 | WP_003372387 | 5 | 1-423 | 424-846 | 847-1069 | 1070 -1252 |
| BoNT/F1 | Q57236 | 6 | 1-439 | 440-865 | 866-1087 | 1088-1278 |
| BoNT/F7 | UPI0001DE3DAC | 11 | 1-508 | 509-862 | 863-1076 | 1077-1268 |
| BoNT/G | WP_039635782 | 7 | 1-446 | 447-864 | 865-1089 | 1090-1297 |
| BoNT/DC | BAM65681 | 8 | 1-442 | 443-863 | 864-1091 | 1092-1285 |

The above-identified reference sequences should be considered a guide, as slight variations may occur according to sub-serotypes. By way of example, US 2007/0166332 cites slightly different clostridial sequences.

The term "activation loop" refers to a polypeptide domain comprising a proteolytic cleavage site. Activation loops of neurotoxins have been described in the art, such as in WO2016156113.

In one embodiment of the invention, the H_{CC} domain of the clostridial neurotoxin consists of or comprises an amino acid sequence selected from the group consisting of:
- amino acid residues 1082 to 1291 of SEQ ID NO: 2, or a sequence having at least 70 %, preferably at least 75%, 80%, 85%, 90%, 95% or 99% sequence identity thereto,
- amino acid residues 1083 to 1276 of SEQ ID NO: 4, or a sequence having at least 70 %, preferably at least 75%, 80%, 85%, 90%, 95% or 99% sequence identity thereto,
- amino acid residues 1088 to 1278 of SEQ ID NO: 6, or a sequence having at least 70 %, preferably at least 75%, 80%, 85%, 90%, 95% or 99% sequence identity thereto,
- amino acid residues 1077 to 1268 of SEQ ID NO: 11, or a sequence having at least 70 %, preferably at least 75%, 80%, 85%, 90%, 95% or 99% sequence identity thereto,
- amino acid residues 1090 to 1297 of SEQ ID NO: 7, or a sequence having at least 70 %, preferably at least 75%, 80%, 85%, 90%, 95% or 99% sequence identity thereto, and
- amino acid residues 1092 to 1285 of SEQ ID NO: 8, or a sequence having at least 70 %, preferably at least 75%, 80%, 85%, 90%, 95% or 99% sequence identity thereto.

As used throughout the specification, the term "percent sequence identity" between two or more amino acid sequences means a function of the number of identical amino acids at identical positions shared by the aligned amino acid sequences. Thus, % identity as used herein may be calculated as the number of identical amino acids at each position in an alignment divided by the total number of amino acids in the aligned sequence, multiplied by 100. Calculations of % sequence identity may also take into account the number of gaps, and the length of each gap that needs to be introduced to optimize alignment of two or more sequences. Sequence comparisons and the determination of percent identity between two or more sequences can be carried out using specific mathematical algorithms, in particular a global alignment mathematical algorithm (such as described by Needleman and Wunsch, J. Mol. Biol. 48(3), 443-453, 1972), which will be familiar to a skilled person.

In a preferred embodiment, the H_{CC} domain consists of or comprises an amino acid sequence selected from the group consisting of:
- amino acid residues 1082 to 1291 of SEQ ID NO: 2, or a sequence having at least 70 %, preferably at least 75%, 80%, 85%, 90%, 95% or 99% sequence identity thereto,
- amino acid residues 1088 to 1278 of SEQ ID NO: 6, or a sequence having at least 70 %, preferably at least 75%, 80%, 85%, 90%, 95% or 99% sequence identity thereto, and
- amino acid residues 1077 to 1268 of SEQ ID NO: 11, or a sequence having at least 70 %, preferably at least 75%, 80%, 85%, 90%, 95% or 99% sequence identity thereto.

In a preferred embodiment, the H_{CC} domain consists of or comprises an amino acid sequence corresponding to amino acid residues 1082 to 1291 of SEQ ID NO: 2, or an amino acid sequence having at least 70 %, preferably at least 75%, 80%, 85%, 90%, 95% or 99% sequence identity thereto.

In a preferred embodiment, the H_{CC} domain consists of or comprises an amino acid sequence corresponding to amino acid residues 1088 to 1278 of SEQ ID NO: 6, or an amino acid sequence having at least 70 %, preferably at least 75%, 80%, 85%, 90%, 95% or 99% sequence identity thereto.

In a preferred embodiment, the H_{CC} domain consists of or comprises an amino acid sequence corresponding to amino acid residues 1077 to 1268 of SEQ ID NO: 11, or an amino acid sequence having at least 70 %, preferably at least 75%, 80%, 85%, 90%, 95% or 99% sequence identity thereto.

In one embodiment, the clostridial neurotoxin consists of or comprises an amino acid sequence having at least 70 %, preferably at least 75%, 80%, 85%, 90%, 95%, 99% or 100% sequence identity to any of SEQ ID NO: 2, SEQ ID NO: 4, SEQ ID NO: 6, SEQ ID NO: 7, SEQ ID NO: 8, SEQ ID NO: 11.

In a preferred embodiment, the clostridial neurotoxin consists of or comprises an amino acid sequence having at least 70 %, preferably at least 75%, 80%, 85%, 90%, 95%, 99% or 100% sequence identity to any of SEQ ID NO: 2, SEQ ID NO: 6, SEQ ID NO: 11.

In a preferred embodiment, the clostridial neurotoxin consists of or comprises an amino acid sequence having at least 70 %, preferably at least 75%, 80%, 85%, 90%, 95%, 99% or 100% sequence identity to SEQ ID NO: 2.

In a preferred embodiment, the clostridial neurotoxin consists of or comprises an amino acid sequence having at least 70 %, preferably at least 75%, 80%, 85%, 90%, 95%, 99% or 100% sequence identity to SEQ ID NO: 6.

In a preferred embodiment, the clostridial neurotoxin consists of or comprises an amino acid sequence having at least 70 %, preferably at least 75%, 80%, 85%, 90%, 95%, 99% or 100% sequence identity to SEQ ID NO: 11.

In a more preferred embodiment, the clostridial neurotoxin is a BoNT/B neurotoxin. Preferably, the BoNT/B clostridial neurotoxin consists of or comprises an amino acid sequence having at least 70 %, preferably at least 75%, 80%, 85%, 90%, 95%, 99% or 100% sequence identity to SEQ ID NO: 2.

In a more preferred embodiment, the clostridial neurotoxin is a BoNT/F neurotoxin. Preferably, the BoNT/F clostridial neurotoxin consists of or comprises an amino acid sequence having at least 70 %, preferably at least 75%, 80%, 85%, 90%, 95%, 99% or 100% sequence identity to SEQ ID NO: 6 or to SEQ ID NO: 11.

In one embodiment, the clostridial neurotoxin is a chimeric neurotoxin.

The term "chimeric neurotoxin" as used herein means a neurotoxin comprising one or more domains originating from a first neurotoxin and one or more domains originating from a second neurotoxin. For example, a chimeric neurotoxin may comprise an LH_{N} domain originating from a first neurotoxin serotype or subtype and a H_{C} domain originating from a second neurotoxin serotype or subtype. Another example of a chimeric neurotoxin is a neurotoxin comprising an LH_{N}H_{CN} domain originating from a first neurotoxin serotype or subtype and a H_{CC} domain originating from a second neurotoxin serotype or subtype. A further example of a chimeric neurotoxin is a neurotoxin comprising an LH_{N} domain from a first neurotoxin serotype or subtype and an activation loop from a second neurotoxin serotype or subtype. Examples of chimeric neurotoxins are provided in WO2017191315 and WO2016156113.

In one embodiment, the clostridial neurotoxin is a chimeric neurotoxin which comprises an H_{C} domain from a BoNT/B and an LH_{N} domain from a BoNT/A, BoNT/C, BoNT/D, BoNT/E, BoNT/F, BoNT/G or BoNT/D-C.

In one embodiment of a chimeric neurotoxin according to the invention, the H_{C} domain consists of or comprises an amino acid sequence selected from the group consisting of:
- amino acid residues 860 to 1291 of SEQ ID NO: 2, or a sequence having at least 70 %, preferably at least 75%, 80%, 85%, 90%, 95% or 99% sequence identity thereto,
- amino acid residues 864 to 1276 of SEQ ID NO: 4, or a sequence having at least 70 %, preferably at least 75%, 80%, 85%, 90%, 95% or 99% sequence identity thereto,
- amino acid residues 866 to 1278 of SEQ ID NO: 6, or a sequence having at least 70 %, preferably at least 75%, 80%, 85%, 90%, 95% or 99% sequence identity thereto,
- amino acid residues 863 to 1268 of SEQ ID NO: 11, or a sequence having at least 70 %, preferably at least 75%, 80%, 85%, 90%, 95% or 99% sequence identity thereto,
- amino acid residues 865 to 1297 of SEQ ID NO: 7, or a sequence having at least 70 %, preferably at least 75%, 80%, 85%, 90%, 95% or 99% sequence identity thereto, and
- amino acid residues 864 to 1285 of SEQ ID NO: 8, or a sequence having at least 70 %, preferably at least 75%, 80%, 85%, 90%, 95% or 99% sequence identity thereto,
and the LH_{N} domain consists of or comprises an amino acid sequence selected from the group consisting of:
- amino acid residues 1 to 872 of SEQ ID NO: 1, or a sequence having at least 70 %, preferably at least 75%, 80%, 85%, 90%, 95% or 99% sequence identity thereto,
- amino acid residues 1 to 859 of SEQ ID NO: 2, or a sequence having at least 70 %, preferably at least 75%, 80%, 85%, 90%, 95% or 99% sequence identity thereto,
- amino acid residues 1 to 867 of SEQ ID NO: 3, or a sequence having at least 70 %, preferably at least 75%, 80%, 85%, 90%, 95% or 99% sequence identity thereto,
- amino acid residues 1 to 863 of SEQ ID NO: 4, or a sequence having at least 70 %, preferably at least 75%, 80%, 85%, 90%, 95% or 99% sequence identity thereto,
- amino acid residues 1 to 846 of SEQ ID NO: 5, or a sequence having at least 70 %, preferably at least 75%, 80%, 85%, 90%, 95% or 99% sequence identity thereto,
- amino acid residues 1 to 865 of SEQ ID NO: 6, or a sequence having at least 70 %, preferably at least 75%, 80%, 85%, 90%, 95% or 99% sequence identity thereto,
- amino acid residues 1 to 862 of SEQ ID NO: 11, or a sequence having at least 70 %, preferably at least 75%, 80%, 85%, 90%, 95% or 99% sequence identity thereto,
- amino acid residues 1 to 864 of SEQ ID NO: 7, or a sequence having at least 70 %, preferably at least 75%, 80%, 85%, 90%, 95% or 99% sequence identity thereto, and
- amino acid residues 1 to 863 of SEQ ID NO: 8, or a polypeptide sequence having at least 70 %, preferably at least 75%, 80%, 85%, 90%, 95% or 99% sequence identity thereto.

In a preferred embodiment, the H_{C} domain consists of or comprises an amino acid sequence corresponding to amino acid residues 860 to 1291 of SEQ ID NO: 2, or an amino acid sequence having at least 70 %, preferably at least 75%, 80%, 85%, 90%, 95% or 99% sequence identity thereto, and the LH_{N} domain consists of or comprises an amino acid sequence selected from the group consisting of:
- amino acid residues 1 to 872 of SEQ ID NO: 1, or a sequence having at least 70 %, preferably at least 75%, 80%, 85%, 90%, 95% or 99% sequence identity thereto,
- amino acid residues 1 to 867 of SEQ ID NO: 3, or a sequence having at least 70 %, preferably at least 75%, 80%, 85%, 90%, 95% or 99% sequence identity thereto,
- amino acid residues 1 to 863 of SEQ ID NO: 4, or a sequence having at least 70 %, preferably at least 75%, 80%, 85%, 90%, 95% or 99% sequence identity thereto,
- amino acid residues 1 to 846 of SEQ ID NO: 5, or a sequence having at least 70 %, preferably at least 75%, 80%, 85%, 90%, 95% or 99% sequence identity thereto,
- amino acid residues 1 to 865 of SEQ ID NO: 6, or a sequence having at least 70 %, preferably at least 75%, 80%, 85%, 90%, 95% or 99% sequence identity thereto,
- amino acid residues 1 to 862 of SEQ ID NO: 11, or a sequence having at least 70 %, preferably at least 75%, 80%, 85%, 90%, 95% or 99% sequence identity thereto,
- amino acid residues 1 to 864 of SEQ ID NO: 7, or a sequence having at least 70 %, preferably at least 75%, 80%, 85%, 90%, 95% or 99% sequence identity thereto, and
- amino acid residues 1 to 863 of SEQ ID NO: 8, or a sequence having at least 70 %, preferably at least 75%, 80%, 85%, 90%, 95% or 99% sequence identity thereto.

In a more preferred embodiment, the clostridial neurotoxin is a chimeric neurotoxin which comprises an H_{C} domain from a BoNT/B and an LH_{N} domain from a BoNT/A.

In a more preferred embodiment, the H_{C} domain consists of or comprises an amino acid sequence corresponding to amino acid residues 860 to 1291 of SEQ ID NO: 2, or an amino acid sequence having at least 70 %, preferably at least 75%, 80%, 85%, 90%, 95% or 99% sequence identity thereto, and the LH_{N} domain comprises an amino acid sequence corresponding to amino acid residues 1 to 872 of SEQ ID NO: 1, or an amino acid sequence having at least 70 %, preferably at least 75%, 80%, 85%, 90%, 95% or 99% sequence identity thereto.

In one embodiment, the clostridial neurotoxin is a chimeric neurotoxin which comprises an H_{CC} domain from a BoNT/B and an LH_{N}H_{CN} domain from a BoNT/A, BoNT/C, BoNT/D, BoNT/E, BoNT/F, BoNT/G or BoNT/D-C.

In one embodiment of a chimeric neurotoxin according to the invention, the H_{CC} domain consists of or comprises an amino acid sequence selected from the group consisting of:
- amino acid residues 1082 to 1291 of SEQ ID NO: 2, or a sequence having at least 70 %, preferably at least 75%, 80%, 85%, 90%, 95% or 99% sequence identity thereto,
- amino acid residues 1083 to 1276 of SEQ ID NO: 4, or a sequence having at least 70 %, preferably at least 75%, 80%, 85%, 90%, 95% or 99% sequence identity thereto,
- amino acid residues 1088 to 1278 of SEQ ID NO: 6, or a sequence having at least 70 %, preferably at least 75%, 80%, 85%, 90%, 95% or 99% sequence identity thereto,
- amino acid residues 1077 to 1268 of SEQ ID NO: 11, or a sequence having at least 70 %, preferably at least 75%, 80%, 85%, 90%, 95% or 99% sequence identity thereto,
- amino acid residues 1090 to 1297 of SEQ ID NO: 7, or a sequence having at least 70 %, preferably at least 75%, 80%, 85%, 90%, 95% or 99% sequence identity thereto, and
- amino acid residues 1092 to 1285 of SEQ ID NO: 8, or a sequence having at least 70 %, preferably at least 75%, 80%, 85%, 90%, 95% or 99% sequence identity thereto,
and the LH_{N}H_{CN} domain consists of or comprises an amino acid sequence selected from the group consisting of:
- amino acid residues 1 to 1094 of SEQ ID NO: 1, or a sequence having at least 70 %, preferably at least 75%, 80%, 85%, 90%, 95% or 99% sequence identity thereto,
- amino acid residues 1 to 1081 of SEQ ID NO: 2, or a sequence having at least 70 %, preferably at least 75%, 80%, 85%, 90%, 95% or 99% sequence identity thereto,
- amino acid residues 1 to 1095 of SEQ ID NO: 3, or a sequence having at least 70 %, preferably at least 75%, 80%, 85%, 90%, 95% or 99% sequence identity thereto,
- amino acid residues 1 to 1082 of SEQ ID NO: 4, or a sequence having at least 70 %, preferably at least 75%, 80%, 85%, 90%, 95% or 99% sequence identity thereto,
- amino acid residues 1 to 1069 of SEQ ID NO: 5, or a sequence having at least 70 %, preferably at least 75%, 80%, 85%, 90%, 95% or 99% sequence identity thereto,
- amino acid residues 1 to 1087 of SEQ ID NO: 6, or a sequence having at least 70 %, preferably at least 75%, 80%, 85%, 90%, 95% or 99% sequence identity thereto,
- amino acid residues 1 to 1076 of SEQ ID NO: 11, or a sequence having at least 70 %, preferably at least 75%, 80%, 85%, 90%, 95% or 99% sequence identity thereto,
- amino acid residues 1 to 1089 of SEQ ID NO: 7, or a sequence having at least 70 %, preferably at least 75%, 80%, 85%, 90%, 95% or 99% sequence identity thereto, and
- amino acid residues 1 to 1091 of SEQ ID NO: 8, or a sequence having at least 70 %, preferably at least 75%, 80%, 85%, 90%, 95% or 99% sequence identity thereto.

In a preferred embodiment, the H_{CC} domain consists of or comprises an amino acid sequence corresponding to amino acid residues 1082 to 1291 of SEQ ID NO: 2, or an amino acid sequence having at least 70 %, preferably at least 75%, 80%, 85%, 90%, 95% or 99% sequence identity thereto, and the LH_{N}H_{CN} domain consists of or comprises an amino acid sequence selected from the group consisting of:
- amino acid residues 1 to 1094 of SEQ ID NO: 1, or a sequence having at least 70 %, preferably at least 75%, 80%, 85%, 90%, 95% or 99% sequence identity thereto,
- amino acid residues 1 to 1095 of SEQ ID NO: 3, or a sequence having at least 70 %, preferably at least 75%, 80%, 85%, 90%, 95% or 99% sequence identity thereto,
- amino acid residues 1 to 1082 of SEQ ID NO: 4, or a sequence having at least 70 %, preferably at least 75%, 80%, 85%, 90%, 95% or 99% sequence identity thereto,
- amino acid residues 1 to 1069 of SEQ ID NO: 5, or a sequence having at least 70 %, preferably at least 75%, 80%, 85%, 90%, 95% or 99% sequence identity thereto,
- amino acid residues 1 to 1087 of SEQ ID NO: 6, or a sequence having at least 70 %, preferably at least 75%, 80%, 85%, 90%, 95% or 99% sequence identity thereto,
- amino acid residues 1 to 1076 of SEQ ID NO: 11, or a sequence having at least 70 %, preferably at least 75%, 80%, 85%, 90%, 95% or 99% sequence identity thereto,
- amino acid residues 1 to 1089 of SEQ ID NO: 7, or a sequence having at least 70 %, preferably at least 75%, 80%, 85%, 90%, 95% or 99% sequence identity thereto, and
- amino acid residues 1 to 1091 of SEQ ID NO: 8, or a sequence having at least 70 %, preferably at least 75%, 80%, 85%, 90%, 95% or 99% sequence identity thereto.

In one embodiment, the clostridial neurotoxin is a chimeric neurotoxin which comprises an LH_{N} domain from a first BoNT/F subtype and an activation loop from a second BoNT/F subtype, wherein said second BoNT/F subtype differs from said first BoNT/F subtype. Such BoNT/F subtypes and BoNT/F chimeric neurotoxins have been described in the art, such as in WO2016156113.

In a preferred embodiment, said LH_{N} domain is a BoNT/F7 subtype LH_{N} domain and/or said activation loop is a BoNT/F1 subtype activation loop.

In a preferred embodiment, said activation loop comprises (or consists of) the amino acid sequence SEQ ID NO: 14.

In a preferred embodiment, the chimeric neurotoxin comprises (or consists of) the amino acid sequence SEQ ID NO: 13, or an amino acid sequence having at least 70 %, preferably at least 75%, 80%, 85%, 90%, 95% or 99% sequence identity thereto.

The clostridial neurotoxin can be a modified neurotoxin or a derivative thereof, including, but not limited to, those described below. A modified neurotoxin or derivative may contain one or more amino acids that have been modified as compared to the native form of the neurotoxin. Examples of such modification, also known as mutation, include, without limitation, an amino acid residue substitution, addition or deletion. In the context of the present invention, a modified clostridial neurotoxin can, for example, have modified amino acid sequence(s) in one or more domains relative to the native clostridial neurotoxin amino acid sequence. The terms native, unmodified, natural, naturally-occurring or wild-type can be used herein interchangeably.

Such modifications may impact functional aspects of the neurotoxin, for example biological activity or persistence. However, in the context of the present invention, a modified neurotoxin is said to be functional. In other words, the modified neurotoxin of the invention retains the function(s) of a neurotoxin, selected from the ability to bind to a low or high affinity neurotoxin receptor on a target cell, the ability to translocate the endopeptidase portion of the neurotoxin (light chain) into the cell cytoplasm and the ability to cleave a SNARE protein.

A modified neurotoxin according to the invention may have one or more modifications in the amino acid sequence of the heavy chain (such as in the H_{C} domain), wherein said modified heavy chain binds to target nerve cells with a higher or lower affinity than the native neurotoxin. Such modifications in the H_{C} domain can include modifications of amino acid residues in the ganglioside binding site of the H_{CC} domain that can alter binding to the ganglioside of the target nerve cell, and/or modifications of amino acid residues in the protein receptor binding site of the H_{CC} domain that can alter binding to the protein receptor of the target nerve cell. Examples of such modified neurotoxins are described in WO2006027207 and WO2006114308.

In one embodiment of a modified clostridial neurotoxin according to the invention, the H_{CC} domain from a BoNT/B, BoNT/D, BoNT/D-C, BoNT/F or BoNT/G is modified as compared to the natural H_{CC} domain of said BoNT serotypes.

In a preferred embodiment, the H_{CC} domain from a BoNT/B, BoNT/D, BoNT/D-C BoNT/F or BoNT/G neurotoxin comprises at least one amino acid residue mutation which increases the binding affinity of said H_{CC} domain for human Syt II as compared to the natural BoNT/B, BoNT/D, BoNT/F, BoNT/G or BoNT/D-C H_{CC} domain. The Inventors have indeed discovered that such amino acid residue mutation can increase the potency of the neurotoxin towards human smooth muscle.

In a more preferred embodiment, the H_{CC} domain from a BoNT/B neurotoxin comprises at least one amino acid residue mutation which increases the binding affinity of said H_{CC} domain for human Syt II as compared to the natural BoNT/B H_{CC} domain. Still, preferably, said at least one amino acid residue mutation increases the binding affinity of said H_{CC} domain for human Syt II by at least 50% as compared to the natural BoNT/B H_{CC} domain.

Such suitable amino acid residue mutations in the BoNT/B H_{CC} domain have been described in the art in WO2013180799 and WO2016154534.

In particular, said at least one amino acid residue mutation suitable for increasing the binding affinity of the BoNT/B H_{CC} domain for human Syt II by at least 50% as compared to the natural BoNT/B H_{CC} domain is an amino acid residue substitution, addition or deletion selected from the group consisting of: 1118M, 1183M, 1191M, 11911, 1191Q, 1191T, 1199Y, 1199F, 1199L, 1201V, 1191C, 1191V, 1191L, 1191Y, 1199W, 1199E, 1199H, 1178Y, 1178Q, 1178A, 1178S, 1183C, 1183P and any combinations thereof. Preferably, said at least one amino acid residue mutation in the BoNT/B H_{CC} domain consists of two amino acid residue substitutions, additions or deletions selected from the group consisting of: 1191M and 1199L, 1191M and 1199Y, 1191M and 1199F, 1191Q and 1199L, 1191Q and 1199Y, 1191Q and 1199F, 1191M and 1199W, 1191M and 1178Q, 1191C and 1199W, 1191C and 1199Y, 1191C and 1178Q, 1191Q and 1199W, 1191V and 1199W, 1191V and 1199Y, or 1191V and 1178Q. Still preferably, said at least one amino acid residue mutation in the BoNT/B H_{CC} domain consists of the three amino acid residue substitutions, additions or deletions: 1191M, 1199W and 1178Q. More preferably, said at least one amino acid residue mutation in BoNT/B H_{CC} domain consists of the two amino acid residue substitutions, additions or deletions: 1191M and 1199Y.

In a more preferred embodiment, said at least one amino acid residue mutation suitable for increasing the binding affinity of the BoN/B H_{CC} domain for human Syt II by at least 50% as compared to the natural BoNT/B H_{CC} domain is an amino acid residue substitution selected from the group consisting of: V1118M, Y1183M, E1191M, E1191I, E1191Q, E1191T, S1199Y, S1199F, S1199L, S1201V, E1191C, E1191V, E1191L, E1191Y, S1199W, S1199E, S1199H, W1178Y, W1178Q, W1178A, W1178S, Y1183C, Y1183P and any combinations thereof. Preferably, said at least one amino acid residue mutation in the BoNT/B H_{CC} domain consists of two amino acid residue substitutions selected from the group consisting of: E1191M and S1199L, E1191M and S1199Y, E1191M and S1199F, E1191Q and S1199L, E1191Q and S1199Y, E1191Q and S1199F, E1191M and S1199W, E1191M and W1178Q, E1191C and S1199W, E1191C and S1199Y, E1191C and W1178Q, E1191Q and S1199W, E1191V and S1199W, E1191V and S1199Y, or E1191V and W1178Q. Still preferably, said at least one amino acid residue mutation in the BoNT/B H_{CC} domain consists of the three amino acid residue substitutions: E1191M, S1199W and W1178Q. More preferably, said at least one amino acid residue mutation in BoNT/B H_{CC} domain consists of the two amino acid residue substitutions: E1191M and S1199Y.

In a preferred embodiment, the BoNT/B H_{CC} domain to be modified corresponds to amino acid residues 1082 to 1291 of SEQ ID NO: 2 (natural BoNT/B H_{CC} domain), or to an amino acid sequence having at least 70 %, preferably at least 80 %, 85 %, 90 %, 95 % or 99 % sequence identity thereto.

In a more preferred embodiment, the modified clostridial neurotoxin of the present invention comprises (or consists of) the amino acid sequence SEQ ID NO: 9, or an amino acid sequence having at least 70 %, preferably at least 75%, 80%, 85%, 90%, 95% or 99% sequence identity thereto.

In a preferred embodiment, the H_{CC} domain from a BoNT/B, BoNT/D, BoNT/D-C BoNT/F or BoNT/G neurotoxin comprises at least one amino acid residue mutation which increases the binding affinity of said H_{CC} domain for one or more gangliosides as compared to the natural BoNT/B, BoNT/D, BoNT/D-C, BoNT/F or BoNT/G H_{CC} domain.

In a more preferred embodiment, said H_{CC} domain from a BoNT/F comprises at least one amino acid residue mutation increasing the binding affinity of said Hcc domain for one or more gangliosides as compared to the natural BoNT/F H_{CC} domain.

In a preferred embodiment, said gangliosides are selected from GD1a and/or GM1a.

Suitable amino acid residue mutations for increasing binding affinity of a BoNT/F H_{CC} domain for gangliosides, such as GD1a and/or GM1a, as compared to a natural BoNT/F H_{CC} domain, include, but are not limited to, the amino acid mutation 1241K, such as an amino acid substitution, addition or deletion. More preferably, said at least one amino acid residue mutation increasing the binding affinity of said BoNT/F H_{CC} domain as compared to a natural BoNT/F Hcc domain is the amino acid substitution H1241K.

In a preferred embodiment, the BoNT/F H_{CC} domain to be modified corresponds to amino acid residues 1088 to 1278 of SEQ ID NO: 6 or to amino acid residues 1077 to 1268 of SEQ ID NO: 11 (natural BoNT/F H_{CC} domains), or to an amino acid sequence having at least 70 %, preferably at least 80 %, 85 %, 90 %, 95 % or 99 % sequence identity thereto.

In a preferred embodiment, the chimeric clostridial neurotoxin of the present invention comprises (or consists of) the amino acid sequence SEQ ID NO: 12, or an amino acid sequence having at least 70 %, preferably at least 75%, 80%, 85%, 90%, 95% or 99% sequence identity thereto.

In one embodiment, the clostridial neurotoxin of the present invention can be both chimeric and modified, as described above. For example, in a preferred embodiment, the clostridial neurotoxin comprises (or consists of) the amino acid sequence SEQ ID NO: 10, or an amino acid sequence having at least 70 %, preferably at least 75%, 80%, 85%, 90%, 95% or 99% sequence identity thereto.

The clostridial neurotoxin of the present invention can be produced using recombinant technologies. Thus, in one embodiment, the clostridial neurotoxin of the invention is a recombinant clostridial neurotoxin.

In one embodiment, the clostridial neurotoxin is associated with BoNT complexing proteins, also known as non-toxic neurotoxin-associated proteins (NAP). In other words, the clostridial neurotoxin is administered to the human patient in association with, or combined with, BoNT complexing proteins. Hence, in one embodiment the clostridial neurotoxin is complexed with one or more BoNT complexing proteins.

In another embodiment, the clostridial neurotoxin is free of (or not associated with, or combined with) BoNT complexing proteins. In other words, the clostridial neurotoxin is administered to the human patient without being associated with, or combined with, BoNT complexing proteins.

Preferably, the clostridial neurotoxin (e.g. for use as described herein) is part of a pharmaceutical composition together with at least one pharmaceutically acceptable carrier. By "pharmaceutically acceptable carrier", it is meant herein any component that is compatible with the other ingredients of the pharmaceutical composition, in particular with the clostridial neurotoxin, and which is not deleterious to the human patient. The pharmaceutically acceptable carrier can be selected on the basis of the desired route of administration, in accordance with standard pharmaceutical practices, and include, without limitation, excipients, diluents, adjuvants, propellants and salts.

Accordingly, the present invention further relates to a pharmaceutical composition for use in the treatment of an autonomic disorder in a human patient, wherein said composition comprises the clostridial neurotoxin of the invention and at least one pharmaceutically acceptable carrier, and the dose of the clostridial neurotoxin to be administered to the patient is as described above. Also encompassed are corresponding uses and methods of treating an autonomic disorder comprising administering a pharmaceutical composition of the invention to a human patient.

The clostridial neurotoxin of the present invention may be formulated for oral, parenteral (i.e. injection), continuous infusion, inhalation or topical administration. Pharmaceutical compositions suitable for injection may be in the form of solutions, suspensions or emulsions, or dry powders which are dissolved or suspended in a suitable vehicle prior to use.

In the case of a neurotoxin that is to be delivered locally, the neurotoxin may be formulated as a cream (e.g. for topical application), or for sub-dermal injection.

Local delivery means may include an aerosol, or other spray (e.g. a nebuliser). In this regard, an aerosol formulation of a neurotoxin enables delivery to the lungs and/or other nasal and/or bronchial or airway passages.

Clostridial neurotoxins of the invention may also be administered to a patient by intrathecal or epidural injection in the spinal column at the level of the spinal segment involved in the innervation of an affected organ.

In one embodiment, the clostridial neurotoxin or pharmaceutical composition is for intramuscular, intradermal or subcutaneous administration.

In one embodiment, the clostridial neurotoxin or pharmaceutical composition is for topical administration, for example by instillation.

A preferred route of administration is via intramuscular injection.

It is nevertheless also possible to administer the clostridial neurotoxin to a muscle without using any injection. For example, to treat urinary disorders, the clostridial neurotoxin may be administered to the bladder by infusing the patients' bladder with a liquid or semi-solid formulation of the clostridial neurotoxin; depositing a gel formulation containing the clostridial neurotoxin at the appropriate location of the patients' bladder; spraying a spray formulation containing the clostridial neurotoxin at the appropriate location of the patients' bladder; or topically applying a solid (e.g. lyophilised), semi-solid or liquid botulinum toxin formulation that is put or spread on the outer walls of a balloon which is then inflated in the bladder so as to be in contact with said bladder's wall, as described in WO2005053733.

As indicated above, the dose of the clostridial neurotoxin which is suitable to achieve the desired therapeutic effect in a human patient suffering from an autonomic disorder, or, in other words, the therapeutic dose of said clostridial neurotoxin, is equivalent to or lower than the dose of BoNT/A treating the same autonomic disorder.

Preferably, said BoNT/A treating the same autonomic disorder is a purified BoNT/A. As used herein, the term "purified BoNT/A" means a botulinum neurotoxin type A purified from a clostridial strain which naturally produces it (naturally-occurring clostridial strain). The purified BoNT/A may be associated with complexing proteins or free of complexing proteins. Examples of commercially available purified BoNT/A include Botox^{®}, Dysport^{®} and Xeomin^{®}.

In one embodiment, the dose of the clostridial neurotoxin to be administered for treating an autonomic disorder in a human patient (therapeutic dose) is about 1.1 times to about 100 times, preferably about 1.3 times to about 90 times, lower than the dose of BoNT/A treating said autonomic disorder.

In a preferred embodiment, when the clostridial neurotoxin comprises a H_{CC} domain from a BoNT/B, the therapeutic dose of said clostridial neurotoxin is about 1.1 times to about 20 times, preferably about 1.3 times to about 19.8 times, lower than the dose of BoNT/A treating the same autonomic disorder. For example, when the clostridial neurotoxin of the invention is a natural BoNT/B neurotoxin, such as the neurotoxin of amino acid sequence SEQ ID NO: 2, the therapeutic dose of said clostridial neurotoxin is about 1.1 times to about 1.5 times, preferably about 1.3 times, lower than the dose of BoNT/A treating the same autonomic disorder. As a further example, when the clostridial neurotoxin of the invention is a modified BoNT/B neurotoxin having an increased binding affinity for the human Sytll receptor (as compared to natural BoNT/B), such as the neurotoxin of amino acid sequence SEQ ID NO: 9, the therapeutic dose of said clostridial neurotoxin is about 15 times to about 20 times, preferably about 19.8 times, lower than the dose of BoNT/A treating the same autonomic disorder. Still, as another example, when the clostridial neurotoxin of the invention is a modified chimeric BoNT/B neurotoxin having an increased binding affinity for the human Sytll receptor, such as the neurotoxin of amino acid sequence SEQ ID NO: 10, the therapeutic dose of said clostridial neurotoxin is about 2 times to about 5 times, preferably about 3.5 times, lower than the dose of BoNT/A treating the same autonomic disorder.

In a preferred embodiment, when the clostridial neurotoxin comprises a H_{CC} domain from a BoNT/F, the therapeutic dose of said clostridial neurotoxin is about 60 times to about 100 times, preferably about 61.2 times to about 90 times, lower than the dose of BoNT/A treating the same autonomic disorder. For example, when the clostridial neurotoxin of the invention is a natural BoNT/F neurotoxin, such as the neurotoxin of amino acid sequence SEQ ID NO: 6 or SEQ ID NO: 11, the therapeutic dose of said clostridial neurotoxin is about 60 times to about 65 times, preferably about 61.2 times, lower than the dose of BoNT/A treating the same autonomic disorder. As a further example, when the clostridial neurotoxin of the invention is a modified BoNT/F neurotoxin having an increased binding affinity for one or more gangliosides (as compared to natural BoNT/F), such as the neurotoxin of amino acid sequence SEQ ID NO: 12, the therapeutic dose of said clostridial neurotoxin is about 60 times to about 65 times, preferably about 61.2 times, lower than the dose of BoNT/A treating the same autonomic disorder. Still, as another example, when the clostridial neurotoxin of the invention is a chimeric BoNT/F neurotoxin, such as the neurotoxin of amino acid sequence SEQ ID NO: 13, the therapeutic dose of said clostridial neurotoxin is about 85 times to about 100 times, preferably about 90 times, lower than the dose of BoNT/A treating the same autonomic disorder.

As indicated above, doses of BoNT/A treating autonomic disorders are well-known in the art (https://www.medicines.org.uk/emc/medicine/112).

The doses of clostridial neurotoxin are herein preferably measured in nanograms.

Doses of clostridial neurotoxin according to the invention are to be understood as doses of active di-chain clostridial neurotoxin, i.e. without including the quantity of complexing proteins to which the neurotoxin may be associated with. In other words, it refers to the doses of active di-chain clostridial neurotoxin, whether said neurotoxin is administered to the patient in association to, or without, complexing proteins. As well-known to the skilled practitioner, an active di-chain clostridial neurotoxin is capable of binding to a neuronal receptor, translocating the light chain into the cytoplasm and of cleaving a SNARE protein, while complexing proteins do not display such biological activity (i.e. are not "active"). In the case of botulinum neurotoxins, the total size of the active di-chain is generally of about 150 kD.

Indeed, as well known to the skilled practitioner, the potency of a clostridial neurotoxin is related to the quantity (e.g. nanograms) of neurotoxin required to achieve an LD50 (lethal dose 50) unit; one LD50 unit being defined as the median lethal intraperitoneal dose (as measured in mice). However, BoNT pharmaceutical preparations currently on the market contain different amount of 150 kD neurotoxin, but also of LD50 Units. Besides, in these preparations, the neurotoxin may, or may not, be associated with (i.e. combined with) non-toxic neurotoxin-associated proteins (NAP), also known as complexing proteins. For ease of conversion:
- 100 Units of Botox^{®} (also known as OnabotulinumtoxinA) contains about 0.73 ng of 150 kD BoNT/A, as well as complexing proteins;
- 100 Units of Dysport^{®} (also known as AbobotulinumtoxinA) contains about 0.65 ng of 150 kD BoNT/A, as well as complexing proteins;
- 100 Units of Xeomin^{®} (also known as IncobotulinumtoxinA) contains about 0.44 ng of 150 kD BoNT/A, with no complexing proteins;
- 100 Units of Neurobloc/Myobloc^{®} (also known as RimabotulinumtoxinB) contains about 0.2 ng to about 1 ng of 150 kD BoNT/B, as well as complexing proteins.

The quantity of clostridial neurotoxin can be measured by the skilled practitioner according to methods conventionally used in the art to quantify proteins preferably at nanograms levels, including, among others, mass spectroscopy such as isotopic dilution mass spectroscopy (Muñoz et al., Quantification of protein calibrants by amino acid analysis using isotope dilution mass spectrometry, Anal. Biochem. 2011, 408, 124-131), or fluorimetric assay (Poras et al., Detection and Quantification of Botulinum Neurotoxin Type A by a Novel Rapid In Vitro Fluorimetric Assay, Appl Environ Microbiol. 2009 Jul; 75(13): 4382-4390).

Where a range of values is herein provided, it shall be understood that, unless the context clearly dictates otherwise, each intervening value to the tenth of the unit between the upper and lower limits of that range is also specifically disclosed. Each smaller range between any stated value or intervening value in a stated range and any other stated or intervening value in that stated range is encompassed within this disclosure. It shall be further understood that any range of numerical values denoted herein by the expression "from a to b" means the range of numerical values extending from a to b (i.e. including the strict end points a and b).

Besides, the term "about" shall be understood herein as plus or minus (±) 5%, preferably ± 4%, ± 3%, ± 2%, ± 1%, ± 0.5%, ± 0.1%, of the numerical value of the number with which it is being used.

The dose of the clostridial neurotoxin of the invention to be administered for treating an autonomic disorder in a human patient (i.e. therapeutic dose) ranges from 0.00025 ng to 3 ng.

In a preferred embodiment, the therapeutic dose of the clostridial neurotoxin is ranging from about 0.0003 ng to about 2 ng, preferably from about 0.0004 ng to about 1.5 ng, from about 0.0005 ng to about 1 ng, still preferably from about 0.0006 ng to about 0.5 ng of said clostridial neurotoxin.

In a preferred embodiment, the clostridial neurotoxin comprises a BoNT/B H_{CC} domain or a BoNT/F H_{CC} domain, and is to be administered to the human patient at any of the dose described herein of said clostridial neurotoxin, for use in the treatment of an autonomic disorder, as described above.

In a more preferred embodiment, the clostridial neurotoxin comprises a BoNT/B H_{CC} domain or a BoNT/F H_{CC} domain, and is to be administered to the human patient at any of the dose described herein of said clostridial neurotoxin, for use in the treatment of a smooth muscle disorder, as described above. More preferably, said smooth muscle disorder is selected from the group consisting of urinary disorders, gastrointestinal disorders, vascular and cardiovascular disorders, prostate disorders and sexual disorders.

For example, the therapeutic dose of the clostridial neurotoxin comprising a BoNT/B H_{CC} domain is preferably ranging from about 0.001 ng to about 2 ng.

Yet, for example, the therapeutic dose of the clostridial neurotoxin comprising a BoNT/B H_{CC} domain is preferably ranging from about 0.0003 ng to about 0.05 ng.

It will nevertheless be appreciated that the dose range required depends on the precise nature of the clostridial neurotoxin, the autonomic disorder, the route of administration, the nature of the formulation, the age of the patient, the nature, extent or severity of the patient's condition, contraindications, if any, and the judgement of the attending physician. Variations in these dosage levels can be adjusted using standard empirical routines for optimisation.

As a reference, examples of suitable doses of natural BoNT/A (such as the neurotoxin having the amino acid sequence SEQ ID NO: 1), for treating some specific autonomic disorders, are provided below:
- NDO (neurogenic detrusor overactivity): 1.46 ng of natural BoNT/A into the detrusor muscle;
- OAB (overactive bladder): 0.73 ng of natural BoNT/A into the detrusor muscle;
- hyperhidrosis of the axillae: 0.365 ng of natural BoNT/A into each axilla;
- sialorrhea in adult patients, in particular in Parkinson Disease patients: 0.146 ng of natural BoNT/A per submandibular and/or parotid gland;
- sialorrhea in pediatric cerebral palsy patients: 0.073 ng of natural BoNT/A per submandibular and/or parotid gland.

Examples of suitable dose ranges of natural BoNT/B (such as the neurotoxin having the amino acid sequence SEQ ID NO: 2), for treating the above-mentioned disorders according to the invention, are:
- NDO (neurogenic detrusor overactivity): 0.5 ng to 2 ng of natural BoNT/B into the detrusor muscle;
- OAB (overactive bladder): 0.25 ng to 1 ng of natural BoNT/B into the detrusor muscle;
- hyperhidrosis of the axillae: 0.125 ng to 0.5 ng of natural BoNT/B into each axilla;
- sialorrhea in adult patients, in particular in Parkinson Disease patients: 0.05 ng to 0.2 ng of natural BoNT/B per submandibular and/or parotid gland;
- sialorrhea in pediatric cerebral palsy patients: 0.025 ng to 0.1 ng of natural BoNT/B per submandibular and/or parotid gland.

Examples of suitable dose ranges of modified BoNT/B having an increased binding affinity for the human Sytll receptor (such as the neurotoxin having the amino acid sequence SEQ ID NO: 9), for treating the above-mentioned disorders according to the invention, are:
- NDO (neurogenic detrusor overactivity): 0.025 ng to 0.2 ng of modified BoNT/B into the detrusor muscle;
- OAB (overactive bladder): 0.0125 ng to 0.1 ng of modified BoNT/B into the detrusor muscle;
- hyperhidrosis of the axillae: 0.0075 ng to 0.05 ng of modified BoNT/B into each axilla;
- sialorrhea in adult patients, in particular Parkinson Disease patients: 0.0025 ng to 0.02 ng of modified BoNT/B per submandibular and/or parotid gland;
- sialorrhea in pediatric cerebral palsy patients: 0.001 ng to 0.01 ng of modified BoNT/B per submandibular and/or parotid gland.

Examples of suitable dose ranges of chimeric modified BoNT/B having an increased binding affinity for the human Sytll receptor (such as the neurotoxin having the amino acid sequence SEQ ID NO: 10), for treating the above-mentioned disorders according to the invention, are:
- NDO (neurogenic detrusor overactivity): 0.2 ng to 1 ng of chimeric modified BoNT/B into the detrusor muscle;
- OAB (overactive bladder): 0.1 ng to 0.5 ng of chimeric modified BoNT/B into the detrusor muscle;
- hyperhidrosis of the axillae: 0.05 ng to 0.2 ng of chimeric modified BoNT/B into each axilla;
- sialorrhea in adult patients, in particular Parkinson Disease patients: 0.02 ng to 0.1 ng of chimeric modified BoNT/B per submandibular and/or parotid gland;
- sialorrhea in pediatric cerebral palsy patients: 0.01 ng to 0.05 ng of chimeric modified BoNT/B per submandibular and/or parotid gland.

Examples of suitable dose ranges of natural BoNT/F (such as the neurotoxin having the amino acid sequence SEQ ID NO: 6 or SEQ ID NO: 11), for treating the above-mentioned disorders according to the invention, are:
- NDO (neurogenic detrusor overactivity): 0.01 ng to 0.05 ng of natural BoNT/F into the detrusor muscle;
- OAB (overactive bladder): 0.005 ng to 0.025 ng of natural BoNT/F into the detrusor muscle;
- hyperhidrosis of the axillae: 0.0025 ng to 0.01 ng of natural BoNT/F into each axilla;
- sialorrhea in adult patients, in particular Parkinson Disease patients: 0.001 ng to 0.005 ng of natural BoNT/F per submandibular and/or parotid gland;
- sialorrhea in pediatric cerebral palsy patients: 0.0005 ng to 0.0025 ng of natural BoNT/F per submandibular and/or parotid gland.

Examples of suitable dose ranges of modified BoNT/F having an increased binding affinity for one or more gangliosides (such as the neurotoxin having the amino acid sequence SEQ ID NO: 12, for treating the above-mentioned disorders according to the invention, are:
- NDO (neurogenic detrusor overactivity): 0.01 ng to 0.05 ng of modified BoNT/F into the detrusor muscle;
- OAB (overactive bladder): 0.005 ng to 0.025 ng of modified BoNT/F into the detrusor muscle;
- hyperhidrosis of the axillae: 0.0025 ng to 0.01 ng of modified BoNT/F into each axilla;
- sialorrhea in adult patients, in particular Parkinson Disease patients: 0.001 ng to 0.005 ng of modified BoNT/F per submandibular and/or parotid gland;
- sialorrhea in pediatric cerebral palsy patients: 0.0005 ng to 0.0025 ng of modified BoNT/F per submandibular and/or parotid gland.

Examples of suitable dose ranges of chimeric BoNT/F (such as the neurotoxin having the amino acid sequence SEQ ID NO: 13), for treating the above-mentioned disorders according to the invention, are:
- NDO (neurogenic detrusor overactivity): 0.007 ng to 0.03 ng of chimeric BoNT/F into the detrusor muscle;
- OAB (overactive bladder): 0.003 ng to 0.0015 ng of chimeric BoNT/F into the detrusor muscle;
- hyperhidrosis of the axillae: 0.001 ng to 0.007 ng of chimeric BoNT/F into each axilla;
- sialorrhea in adult patients, in particular Parkinson Disease patients: 0.0007 ng to 0.003 ng of chimeric BoNT/F per submandibular and/or parotid gland;
- sialorrhea in pediatric cerebral palsy patients: 0.0003 ng to 0.0015 ng of chimeric BoNT/F per submandibular and/or parotid gland.

This disclosure is not limited by the exemplary methods and materials disclosed herein, and any methods and materials similar or equivalent to those described herein can be used in the practice or testing of embodiments of this disclosure. Unless otherwise indicated, any amino acid sequence is written left to right in amino to carboxy orientation, respectively.

It must further be noted that as used herein and in the appended claims, the singular forms "a", "an", and "the" include plural referents unless the context clearly dictates otherwise. Thus, for example, reference to "a clostridial neurotoxin" includes a plurality of such candidate agents and reference to "the clostridial neurotoxin" includes reference to one or more clostridial neurotoxins and equivalents thereof known to those skilled in the art, and so forth.

The invention will now be described, by way of example only, with reference to the following Figures and Examples.

### DESCRIPTION OF THE FIGURES

**Figure 1** - Bladder strip contractile responses to EFS-induced contractions following 3 stimulations applied at 1-min interval followed by 3 min rest.
**Figure 2A** - Time for 50% (T50) inhibition of maximal EFS contraction in terms of amplitude at 1, 3, 5 or 10 nM of nBoNT/A, nBoNT/B, rBoNT/B_{MY} and rBoNT/AB_{MY}.
**Figure 2B** - Time for 50% (T50) inhibition of maximal EFS contraction in terms of amplitude at 0.1, 1 or 10 nM of nBoNT/F, mrBoNT/F, and mrBoNT/F7-1.
**Figure 3** - Concentration response curves of nBoNT/A, nBoNT/B, rBoNT/B_{MY}, rBoNT/AB_{MY,} nBoNT/F, and mrBoNT/F7-1. Time for 50% (T50) inhibition was plotted against the botulinum toxin protein concentration, and logarithmic functions were fitted yielding excellent *R²* values.

### AMINO ACID SEQUENCES

- SEQ ID NO: 1 - BoNT/A1, accession number A5HZZ9, amino acid sequence 
MPFVNKQFNYKDPVNGVDIAYIKIPNAGQMQPVKAFKIHNKIWVIPERDTFTNPEEGDLNPPPEA
KQVPVSYYDSTYLSTDNEKDNYLKGVTKLFERIYSTDLGRMLLTSIVRGIPFWGGSTIDTELKVI
DTNCINVIQPDGSYRSEELNLVIIGPSADIIQFECKSFGHEVLNLTRNGYGSTQYIRFSPDFTFG
FEESLEVDTNPLLGAGKFATDPAVTLAHELIHAGHRLYGIAINPNRVFKVNTNAYYEMSGLEVSF
EELRTFGGHDAKFIDSLQENEFRLYYYNKFKDIASTLNKAKSIVGTTASLQYMKNVFKEKYLLSE
DTSGKFSVDKLKFDKLYKMLTEIYTEDNFVKFFKVLNRKTYLNFDKAVFKINIVPKVNYTIYDGF
NLRNTNLAANFNGQNTEINNMNFTKLKNFTGLFEFYKLLCVRGIITSKTKSLDKGYNKALNDLCI
KVNNWDLFFSPSEDNFTNDLNKGEEITSDTNIEAAEENISLDLIQQYYLTFNFDNEPENISIENL
SSDIIGQLELMPNIERFPNGKKYELDKYTMFHYLRAQEFEHGKSRIALTNSVNEALLNPSRVYTF
FSSDYVKKVNKATEAAMFLGWVEQLVYDFTDETSEVSTTDKIADITIIIPYIGPALNIGNMLYKD
DFVGALIFSGAVILLEFIPEIAIPVLGTFALVSYIANKVLTVQTIDNALSKRNEKWDEVYKYIVT
NWLAKVNTQIDLIRKKMKEALENQAEATKAIINYQYNQYTEEEKNNINFNIDDLSSKLNESINKA
MININKFLNQCSVSYLMNSMIPYGVKRLEDFDASLKDALLKYIYDNRGTLIGQVDRLKDKVNNTL
STDIPFQLSKYVDNQRLLSTFTEYIKNIINTSILNLRYESNHLIDLSRYASKINIGSKVNFDPID
KNQIQLFNLESSKIEVILKNAIVYNSMYENFSTSFWIRIPKYFNSISLNNEYTIINCMENNSGWK
VSLNYGEIIWTLQDTQEIKQRVVFKYSQMINISDYINRWIFVTITNNRLNNSKIYINGRLIDQKP
ISNLGNIHASNNIMFKLDGCRDTHRYIWIKYFNLFDKELNEKEIKDLYDNQSNSGILKDFWGDYL
QYDKPYYMLNLYDPNKYVDVNNVGIRGYMYLKGPRGSVMTTNIYLNSSLYRGTKFIIKKYASGNK
DNIVRNNDRVYINVVVKNKEYRLATNASQAGVEKILSALEIPDVGNLSQVVVMKSKNDQGITNKC
KMNLQDNNGNDIGFIGFHQFNNIAKLVASNWYNRQIERSSRTLGCSWEFIPVDDGWGERPL
- SEQ ID NO: 2 - BoNT/B1, accession number B1INP5, amino acid sequence 
MPVTINNFNYNDPIDNNNIIMMEPPFARGTGRYYKAFKITDRIWIIPERYTFGYKPEDFNKSSGI
FNRDVCEYYDPDYLNTNDKKNIFLQTMIKLFNRIKSKPLGEKLLEMIINGIPYLGDRRVPLEEFN
TNIASVTVNKLISNPGEVERKKGIFANLIIFGPGPVLNENETIDIGIQNHFASREGFGGIMQMKF
CPEYVSVFNNVQENKGASIFNRRGYFSDPALILMHELIHVLHGLYGIKVDDLPIVPNEKKFFMQS
TDAIQAEELYTFGGQDPSIITPSTDKSIYDKVLQNFRGIVDRLNKVLVCISDPNININIYKNKFK
DKYKFVEDSEGKYSIDVESFDKLYKSLMFGFTETNIAENYKIKTRASYFSDSLPPVKIKNLLDNE
IYTIEEGFNISDKDMEKEYRGQNKAINKQAYEEISKEHLAVYKIQMCKSVKAPGICIDVDNEDLF
FIADKNSFSDDLSKNERIEYNTQSNYIENDFPINELILDTDLISKIELPSENTESLTDFNVDVPV
 
YEKQPAIKKIFTDENTIFQYLYSQTFPLDIRDISLTSSFDDALLFSNKVYSFFSMDYIKTANKVV
EAGLFAGWVKQIVNDFVIEANKSNTMDKIADISLIVPYIGLALNVGNETAKGNFENAFEIAGASI
LLEFIPELLIPVVGAFLLESYIDNKNKIIKTIDNALTKRNEKWSDMYGLIVAQWLSTVNTQFYTI
KEGMYKALNYQAQALEEIIKYRYNIYSEKEKSNINIDFNDINSKLNEGINQAIDNINNFINGCSV
SYLMKKMIPLAVEKLLDFDNTLKKNLLNYIDENKLYLIGSAEYEKSKVNKYLKTIMPFDLSIYTN
DTILIEMFNKYNSEILNNIILNLRYKDNNLIDLSGYGAKVEVYDGVELNDKNQFKLTSSANSKIR
VTQNQNIIFNSVFLDFSVSFWIRIPKYKNDGIQNYIHNEYTIINCMKNNSGWKISIRGNRIIWTL
IDINGKTKSVFFEYNIREDISEYINRWFFVTITNNLNNAKIYINGKLESNTDIKDIREVIANGEI
IFKLDGDIDRTQFIWMKYFSIFNTELSQSNIEERYKIQSYSEYLKDFWGNPLMYNKEYYMFNAGN
KNSYIKLKKDSPVGEILTRSKYNQNSKYINYRDLYIGEKFIIRRKSNSQSINDDIVRKEDYIYLD
FFNLNQEWRVYTYKYFKKEEEKLFLAPISDSDEFYNTIQIKEYDEQPTYSCQLLFKKDEESTDEI
GLIGIHRFYESGIVFEEYKDYFCISKWYLKEVKRKPYNLKLGCNWQFIPKDEGWTE
- SEQ ID NO: 3 - BoNT/C1, accession number P18640, amino acid sequence 
MPITINNFNYSDPVDNKNILYLDTHLNTLANEPEKAFRITGNIWVIPDRFSRNSNPNLNKPPRVT
SPKSGYYDPNYLSTDSDKDPFLKEIIKLFKRINSREIGEELIYRLSTDIPFPGNNNTPINTFDFD
VDFNSVDVKTRQGNNWVKTGSINPSVIITGPRENIIDPETSTFKLTNNTFAAQEGFGALSIISIS
PRFMLTYSNATNDVGEGRFSKSEFCMDPILILMHELNHAMHNLYGIAIPNDQTISSVTSNIFYSQ
YNVKLEYAEIYAFGGPTIDLIPKSARKYFEEKALDYYRSIAKRLNSITTANPSSFNKYIGEYKQK
LIRKYRFVVESSGEVTVNRNKFVELYNELTQIFTEFNYAKIYNVQNRKIYLSNVYTPVTANILDD
NVYDIQNGFNIPKSNLNVLFMGQNLSRNPALRKVNPENMLYLFTKFCHKAIDGRSLYNKTLDCRE
LLVKNTDLPFIGDISDVKTDIFLRKDINEETEVIYYPDNVSVDQVILSKNTSEHGQLDLLYPSID
SESEILPGENQVFYDNRTQNVDYLNSYYYLESQKLSDNVEDFTFTRSIEEALDNSAKVYTYFPTL
ANKVNAGVQGGLFLMWANDVVEDFTTNILRKDTLDKISDVSAIIPYIGPALNISNSVRRGNFTEA
FAVTGVTILLEAFPEFTIPALGAFVIYSKVQERNEIIKTIDNCLEQRIKRWKDSYEWMMGTWLSR
IITQFNNISYQMYDSLNYQAGAIKAKIDLEYKKYSGSDKENIKSQVENLKNSLDVKISEAMNNIN
KFIRECSVTYLFKNMLPKVIDELNEFDRNTKAKLINLIDSHNIILVGEVDKLKAKVNNSFQNTIP
FNIFSYTNNSLLKDIINEYFNNINDSKILSLQNRKNTLVDTSGYNAEVSEEGDVQLNPIFPFDFK
LGSSGEDRGKVIVTQNENIVYNSMYESFSISFWIRINKWVSNLPGYTIIDSVKNNSGWSIGIISN
FLVFTLKQNEDSEQSINFSYDISNNAPGYNKWFFVTVTNNMMGNMKIYINGKLIDTIKVKELTGI
NFSKTITFEINKIPDTGLITSDSDNINMWIRDFYIFAKELDGKDINILFNSLQYTNVVKDYWGND
LRYNKEYYMVNIDYLNRYMYANSRQIVFNTRRNNNDFNEGYKIIIKRIRGNTNDTRVRGGDILYF
DMTINNKAYNLFMKNETMYADNHSTEDIYAIGLREQTKDINDNIIFQIQPMNNTYYYASQIFKSN
FNGENISGICSIGTYRFRLGGDWYRHNYLVPTVKQGNYASLLESTSTHWGFVPVSE
- SEQ ID NO: 4 - BoNT/D, accession number P19321, amino acid sequence 
MTWPVKDFNYSDPVNDNDILYLRIPQNKLITTPVKAFMITQNIWVIPERFSSDTNPSLSKPPRPT
SKYQSYYDPSYLSTDEQKDTFLKGIIKLFKRINERDIGKKLINYLVVGSPFMGDSSTPEDTFDFT
RHTTNIAVEKFENGSWKVTNIITPSVLIFGPLPNILDYTASLTLQGQQSNPSFEGFGTLSILKVA
PEFLLTFSDVTSNQSSAVLGKSIFCMDPVIALMHELTHSLHQLYGINIPSDKRIRPQVSEGFFSQ
DGPNVQFEELYTFGGLDVEIIPQIERSQLREKALGHYKDIAKRLNNINKTIPSSWISNIDKYKKI
FSEKYNFDKDNTGNFVVNIDKFNSLYSDLTNVMSEVVYSSQYNVKNRTHYFSRHYLPVFANILDD
NIYTIRDGFNLTNKGFNIENSGQNIERNPALQKLSSESVVDLFTKVCLRLTKNSRDDSTCIKVKN
NRLPYVADKDSISQEIFENKIITDETNVQNYSDKFSLDESILDGQVPINPEIVDPLLPNVNMEPL
NLPGEEIVFYDDITKYVDYLNSYYYLESQKLSNNVENITLTTSVEEALGYSNKIYTFLPSLAEKV
NKGVQAGLFLNWANEVVEDFTTNIMKKDTLDKISDVSVIIPYIGPALNIGNSALRGNFNQAFATA
GVAFLLEGFPEFTIPALGVFTFYSSIQEREKIIKTIENCLEQRVKRWKDSYQWMVSNWLSRITTQ
FNHINYQMYDSLSYQADAIKAKIDLEYKKYSGSDKENIKSQVENLKNSLDVKISEAMNNINKFIR
ECSVTYLFKNMLPKVIDELNKFDLRTKTELINLIDSHNIILVGEVDRLKAKVNESFENTMPFNIF
SYTNNSLLKDIINEYFNSINDSKILSLQNKKNALVDTSGYNAEVRVGDNVQLNTIYTNDFKLSSS
GDKIIVNLNNNILYSAIYENSSVSFWIKISKDLTNSHNEYTIINSIEQNSGWKLCIRNGNIEWIL
QDVNRKYKSLIFDYSESLSHTGYTNKWFFVTITNNIMGYMKLYINGELKQSQKIEDLDEVKLDKT
IVFGIDENIDENQMLWIRDFNIFSKELSNEDINIVYEGQILRNVIKDYWGNPLKFDTEYYIINDN
YIDRYIAPESNVLVLVQYPDRSKLYTGNPITIKSVSDKNPYSRILNGDNIILHMLYNSRKYMIIR
DTDTIYATQGGECSQNCVYALKLQSNLGNYGIGIFSIKNIVSKNKYCSQIFSSFRENTMLLADIY
KPWRFSFKNAYTPVAVTNYETKLLSTSSFWKFISRDPGWVE
- SEQ ID NO: 5 - BoNT/E1, accession number WP 003372387, amino acid sequence 
MPKINSFNYNDPVNDRTILYIKPGGCQEFYKSFNIMKNIWIIPERNVIGTTPQDFHPPTSLKNGD
SSYYDPNYLQSDEEKDRFLKIVTKIFNRINNNLSGGILLEELSKANPYLGNDNTPDNQFHIGDAS
AVEIKFSNGSQDILLPNVIIMGAEPDLFETNSSNISLRNNYMPSNHGFGSIAIVTFSPEYSFRFN
DNSMNEFIQDPALTLMHELIHSLHGLYGAKGITTKYTITQKQNPLITNIRGTNIEEFLTFGGTDL
NIITSAQSNDIYTNLLADYKKIASKLSKVQVSNPLLNPYKDVFEAKYGLDKDASGIYSVNINKFN
DIFKKLYSFTEFDLATKFQVKCRQTYIGQYKYFKLSNLLNDSIYNISEGYNINNLKVNFRGQNAN
LNPRIITPITGRGLVKKIIRFCKNIVSVKGIRKSICIEINNGELFFVASENSYNDDNINTPKEID
DTVTSNNNYENDLDQVILNFNSESAPGLSDEKLNLTIQNDAYIPKYDSNGTSDIEQHDVNELNVF
FYLDAQKVPEGENNVNLTSSIDTALLEQPKIYTFFSSEFINNVNKPVQAALFVSWIQQVLVDFTT
EANQKSTVDKIADISIVVPYIGLALNIGNEAQKGNFKDALELLGAGILLEFEPELLIPTILVFTI
KSFLGSSDNKNKVIKAINNALKERDEKWKEVYSFIVSNWMTKINTQFNKRKEQMYQALQNQVNAI
KTIIESKYNSYTLEEKNELTNKYDIKQIENELNQKVSIAMNNIDRFLTESSISYLMKLINEVKIN
KLREYDENVKTYLLNYIIQHGSILGESQQELNSMVTDTLNNSIPFKLSSYTDDKILISYFNKFFK
RIKSSSVLNMRYKNDKYVDTSGYDSNININGDVYKYPTNKNQFGIYNDKLSEVNISQNDYIIYDN
 
KYKNFSISFWVRIPNYDNKIVNVNNEYTIINCMRDNNSGWKVSLNHNEIIWTLQDNAGINQKLAF
NYGNANGISDYINKWIFVTITNDRLGDSKLYINGNLIDQKSILNLGNIHVSDNILFKIVNCSYTR
YIGIRYFNIFDKELDETEIQTLYSNEPNTNILKDFWGNYLLYDKEYYLLNVLKPNNFIDRRKDST
LSINNIRSTILLANRLYSGIKVKIQRVNNSSTNDNLVRKNDQVYINFVASKTHLFPLYADTATTN
KEKTIKISSSGNRFNQVVVMNSVGNNCTMNFKNNNGNNIGLLGFKADTVVASTWYYTHMRDHTNS
NGCFWNFISEEHGWQEK
- SEQ ID NO: 6 - BoNT/F1, accession number Q57236, amino acid sequence 
MPVVINSFNYNDPVNDDTILYMQIPYEEKSKKYYKAFEIMRNVWIIPERNTIGTDPSDFDPPASL
ENGSSAYYDPNYLTTDAEKDRYLKTTIKLFKRINSNPAGEVLLQEISYAKPYLGNEHTPINEFHP
VTRTTSVNIKSSTNVKSSIILNLLVLGAGPDIFENSSYPVRKLMDSGGVYDPSNDGFGSINIVTF
SPEYEYTFNDISGGYNSSTESFIADPAISLAHELIHALHGLYGARGVTYKETIKVKQAPLMIAEK
PIRLEEFLTFGGQDLNIITSAMKEKIYNNLLANYEKIATRLSRVNSAPPEYDINEYKDYFQWKYG
LDKNADGSYTVNENKFNEIYKKLYSFTEIDLANKFKVKCRNTYFIKYGFLKVPNLLDDDIYTVSE
GFNIGNLAVNNRGQNIKLNPKIIDSIPDKGLVEKIVKFCKSVIPRKGTKAPPRLCIRVNNRELFF
VASESSYNENDINTPKEIDDTTNLNNNYRNNLDEVILDYNSETIPQISNQTLNTLVQDDSYVPRY
DSNGTSEIEEHNVVDLNVFFYLHAQKVPEGETNISLTSSIDTALSEESQVYTFFSSEFINTINKP
VHAALFISWINQVIRDFTTEATQKSTFDKIADISLVVPYVGLALNIGNEVQKENFKEAFELLGAG
ILLEFVPELLIPTILVFTIKSFIGSSENKNKIIKAINNSLMERETKWKEIYSWIVSNWLTRINTQ
FNKRKEQMYQALQNQVDAIKTVIEYKYNNYTSDERNRLESEYNINNIREELNKKVSLAMENIERF
ITESSIFYLMKLINEAKVSKLREYDEGVKEYLLDYISEHRSILGNSVQELNDLVTSTLNNSIPFE
LSSYTNDKILILYFNKLYKKIKDNSILDMRYENNKFIDISGYGSNISINGDVYIYSTNRNQFGIY
SSKPSEVNIAQNNDIIYNGRYQNFSISFWVRIPKYFNKVNLNNEYTIIDCIRNNNSGWKISLNYN
KIIWTLQDTAGNNQKLVFNYTQMISISDYINKWIFVTITNNRLGNSRIYINGNLIDEKSISNLGD
IHVSDNILFKIVGCNDTRYVGIRYFKVFDTELGKTEIETLYSDEPDPSILKDFWGNYLLYNKRYY
LLNLLRTDKSITQNSNFLNINQQRGVYQKPNIFSNTRLYTGVEVIIRKNGSTDISNTDNFVRKND
LAYINVVDRDVEYRLYADISIAKPEKIIKLIRTSNSNNSLGQIIVMDSIGNNCTMNFQNNNGGNI
GLLGFHSNNLVASSWYYNNIRKNTSSNGCFWSFISKEHGWQEN
- SEQ ID NO: 7 - BoNT/G, accession number WP 039635782, amino acid sequence 
MPVNIKNFNYNDPINNDDIIMMEPFNDPGPGTYYKAFRIIDRIWIVPERFTYGFQPDQFNASTGV
FSKDVYEYYDPTYLKTDAEKDKFLKTMIKLFNRINSKPSGQRLLDMIVDAIPYLGNASTPPDKFA
ANVANVSINKKIIQPGAEDQIKGLMTNLIIFGPGPVLSDNFTDSMIMNGHSPISEGFGARMMIRF
CPSCLNVFNNVQENKDTSIFSRRAYFADPALTLMHELIHVLHGLYGIKISNLPITPNTKEFFMQH
SDPVQAEELYTFGGHDPSVISPSTDMNIYNKALQNFQDIANRLNIVSSAQGSGIDISLYKQIYKN
KYDFVEDPNGKYSVDKDKFDKLYKALMFGFTETNLAGEYGIKTRYSYFSEYLPPIKTEKLLDNTI
YTQNEGFNIASKNLKTEFNGQNKAVNKEAYEEISLEHLVIYRIAMCKPVMYKNTGKSEQCIIVNN
 
EDLFFIANKDSFSKDLAKAETIAYNTQNNTIENNFSIDQLILDNDLSSGIDLPNENTEPFTNFDD
IDIPVYIKQSALKKIFVDGDSLFEYLHAQTFPSNIENLQLTNSLNDALRNNNKVYTFFSTNLVEK
ANTVVGASLFVNWVKGVIDDFTSESTQKSTIDKVSDVSIIIPYIGPALNVGNETAKENFKNAFEI
GGAAILMEFIPELIVPIVGFFTLESYVGNKGHIIMTISNALKKRDQKWTDMYGLIVSQWLSTVNT
QFYTIKERMYNALNNQSQAIEKIIEDQYNRYSEEDKMNINIDFNDIDFKLNQSINLAINNIDDFI
NQCSISYLMNRMIPLAVKKLKDFDDNLKRDLLEYIDTNELYLLDEVNILKSKVNRHLKDSIPFDL
SLYTKDTILIQVFNNYISNISSNAILSLSYRGGRLIDSSGYGATMNVGSDVIFNDIGNGQFKLNN
SENSNITAHQSKFVVYDSMFDNFSINFWVRTPKYNNNDIQTYLQNEYTIISCIKNDSGWKVSIKG
NRIIWTLIDVNAKSKSIFFEYSIKDNISDYINKWFSITITNDRLGNANIYINGSLKKSEKILNLD
RINSSNDIDFKLINCTDTTKFVWIKDFNIFGRELNATEVSSLYWIQSSTNTLKDFWGNPLRYDTQ
YYLFNQGMQNIYIKYFSKASMGETAPRTNFNNAAINYQNLYLGLRFIIKKASNSRNINNDNIVRE
GDYIYLNIDNISDESYRVYVLVNSKEIQTQLFLAPINDDPTFYDVLQIKKYYEKTTYNCQILCEK
DTKTFGLFGIGKFVKDYGYVWDTYDNYFCISQWYLRRISENINKLRLGCNWQFIPVDEGWTE
- SEQ ID NO: 8 - BoNT/DC, accession number BAM65681, amino acid sequence 
MTWPVKDFNYSDPVNDNDILYLRIPQNKLITTPVKAFMITQNIWVIPERFSSDTNPSLSKPPRPT
SKYQSYYDPSYLSTDEQKDTFLKGIIKLFKRINERDIGKKLINYLVVGSPFMGDSSTPEDTFDFT
RHTTNIAVEKFENGSWKVTNIITPSVLIFGPLPNILDYTASLTLQGQQSNPSFEGFGTLSILKVA
PEFLLTFSDVTSNQSSAVLGKSIFCMDPVIALMHELTHSLHQLYGINIPSDKRIRPQVSEGFFSQ
DGPNVQFEELYTFGGSDVEIIPQIERLQLREKALGHYKDIAKRLNNINKTIPSSWSSNIDKYKKI
FSEKYNFDKDNTGNFVVNIDKFNSLYSDLTNVMSEVVYSSQYNVKNRTHYFSKHYLPVFANILDD
NIYTIINGFNLTTKGFNIENSGQNIERNPALQKLSSESVVDLFTKVCLRLTRNSRDDSTCIQVKN
NTLPYVADKDSISQEIFESQIITDETNVENYSDNFSLDESILDAKVPTNPEAVDPLLPNVNMEPL
NVPGEEEVFYDDITKDVDYLNSYYYLEAQKLSNNVENITLTTSVEEALGYSNKIYTFLPSLAEKV
NKGVQAGLFLNWANEVVEDFTTNIMKKDTLDKISDVSAIIPYIGPALNIGNSALRGNFKQAFATA
GVAFLLEGFPEFTIPALGVFTFYSSIQEREKIIKTIENCLEQRVKRWKDSYQWMVSNWLSRITTQ
FNHISYQMYDSLSYQADAIKAKIDLEYKKYSGSDKENIKSQVENLKNSLDVKISEAMNNINKFIR
ECSVTYLFKNMLPKVIDELNKFDLKTKTELINLIDSHNIILVGEVDRLKAKVNESFENTIPFNIF
SYTNNSLLKDMINEYFNSINDSKILSLQNKKNTLMDTSGYNAEVRVEGNVQLNPIFPFDFKLGSS
GDDRGKVIVTQNENIVYNAMYESFSISFWIRINKWVSNLPGYTIIDSVKNNSGWSIGIISNFLVF
TLKQNENSEQDINFSYDISKNAAGYNKWFFVTITTNMMGNMMIYINGKLIDTIKVKELTGINFSK
TITFQMNKIPNTGLITSDSDNINMWIRDFYIFAKELDDKDINILFNSLQYTNVVKDYWGNDLRYD
KEYYMINVNYMNRYMSKKGNGIVFNTRKNNNDFNEGYKIIIKRIIGNTNDTRVRGENVLYFNTTI
DNKQYSLGMYKPSRNLGTDLVPLGALDQPMDEIRKYGSFIIQPCNTFDYYASQLFLSSNATTNRI
GILSIGSYSFKLGDDYWFNHEYLIPVIKIEHYASLLESTSTHWVFVPASE
- SEQ ID NO: 9 - BoNT/B_{MY}, amino acid sequence 
MPVTINNFNYNDPIDNNNIIMMEPPFARGTGRYYKAFKITDRIWIIPER 49
YTFGYKPEDFNKSSGIFNRDVCEYYDPDYLNTNDKKNIFLQTMIKLFNRI 99
KSKPLGEKLLEMIINGIPYLGDRRVPLEEFNTNIASVTVNKLISNPGEVE 149
RKKGIFANLIIFGPGPVLNENETIDIGIQNHFASREGFGGIMQMKFCPEY 199
VSVFNNVQENKGASIFNRRGYFSDPALILMHELIHVLHGLYGIKVDDLPI 249
VPNEKKFFMQSTDAIQAEELYTFGGQDPSIITPSTDKSIYDKVLQNFRGI 299
VDRLNKVLVCISDPNININIYKNKFKDKYKFVEDSEGKYSIDVESFDKLY 349
KSLMFGFTETNIAENYKIKTRASYFSDSLPPVKIKNLLDNEIYTIEEGFN 399
ISDKDMEKEYRGQNKAINKQAYEEISKEHLAVYKIQMCKSVKAPGICIDV 449
DNEDLFFIADKNSFSDDLSKNERIEYNTQSNYIENDFPINELILDTDLIS 499
KIELPSENTESLTDFNVDVPVYEKQPAIKKIFTDENTIFQYLYSQTFPLD 549
IRDISLTSSFDDALLFSNKVYSFFSMDYIKTANKVVEAGLFAGWVKQIVN 599
DFVIEANKSNTMDKIADISLIVPYIGLALNVGNETAKGNFENAFEIAGAS 649
ILLEFIPELLIPVVGAFLLESYIDNKNKIIKTIDNALTKRNEKWSDMYGL 699
IVAQWLSTVNTQFYTIKEGMYKALNYQAQALEEIIKYRYNIYSEKEKSNI 749
NIDFNDINSKLNEGINQAIDNINNFINGCSVSYLMKKMIPLAVEKLLDFD 799
NTLKKNLLNYIDENKLYLIGSAEYEKSKVNKYLKTIMPFDLSIYTNDTIL 849
IEMFNKYNSEILNNIILNLRYKDNNLIDLSGYGAKVEVYDGVELNDKNQF 899
KLTSSANSKIRVTQNQNIIFNSVFLDFSVSFWIRIPKYKNDGIQNYIHNE 949
YTIINCMKNNSGWKISIRGNRIIWTLIDINGKTKSVFFEYNIREDISEYI 999
NRWFFVTITNNLNNAKIYINGKLESNTDIKDIREVIANGEIIFKLDGDID 1049
RTQFIWMKYFSIFNTELSQSNIEERYKIQSYSEYLKDFWGNPLMYNKEYY 1099
MFNAGNKNSYIKLKKDSPVGEILTRSKYNQNSKYINYRDLYIGEKFIIRR 1149
KSNSQSINDDIVRKEDYIYLDFFNLNQEWRVYTYKYFKKEEMKLFLAPIY 1199
DSDEFYNTIQIKEYDEQPTYSCQLLFKKDEESTDEIGLIGIHRFYESGIV 1249
FEEYKDYFCISKWYLKEVKRKPYNLKLGCNWQFIPKDEGWTE 1291
- SEQ ID NO: 10 - BoNT/AB_{MY}, amino acid sequence 
MPFVNKQFNYKDPVNGVDIAYIKIPNAGQMQPVKAFKIHNKIWVIPERD 49
TFTNPEEGDLNPPPEAKQVPVSYYDSTYLSTDNEKDNYLKGVTKLFERIY 99
STDLGRMLLTSIVRGIPFWGGSTIDTELKVIDTNCINVIQPDGSYRSEEL 149
NLVIIGPSADIIQFECKSFGHEVLNLTRNGYGSTQYIRFSPDFTFGFEES 199
LEVDTNPLLGAGKFATDPAVTLAHELIHAGHRLYGIAINPNRVFKVNTNA 249
YYEMSGLEVSFEELRTFGGHDAKFIDSLQENEFRLYYYNKFKDIASTLNK 299
AKSIVGTTASLQYMKNVFKEKYLLSEDTSGKFSVDKLKFDKLYKMLTEIY 349
TEDNFVKFFKVLNRKTYLNFDKAVFKINIVPKVNYTIYDGFNLRNTNLAA 399
 
NFNGQNTEINNMNFTKLKNFTGLFEFYKLLCVRGIITSKTKSLDKGYNKA 449
LNDLCIKVNNWDLFFSPSEDNFTNDLNKGEEITSDTNIEAAEENISLDLI 499
QQYYLTFNFDNEPENISIENLSSDIIGQLELMPNIERFPNGKKYELDKYT 549
MFHYLRAQEFEHGKSRIALTNSVNEALLNPSRVYTFFSSDYVKKVNKATE 599
AAMFLGWVEQLVYDFTDETSEVSTTDKIADITIIIPYIGPALNIGNMLYK 649
DDFVGALIFSGAVILLEFIPEIAIPVLGTFALVSYIANKVLTVQTIDNAL 699
SKRNEKWDEVYKYIVTNWLAKVNTQIDLIRKKMKEALENQAEATKAIINY 749
QYNQYTEEEKNNINFNIDDLSSKLNESINKAMININKFLNQCSVSYLMNS 799
MIPYGVKRLEDFDASLKDALLKYIYDNRGTLIGQVDRLKDKVNNTLSTDI 849
PFQLSKYVDNQRLLSTFTEYIKNILNNIILNLRYKDNNLIDLSGYGAKVE 899
VYDGVELNDKNQFKLTSSANSKIRVTQNQNIIFNSVFLDFSVSFWIRIPK 949
YKNDGIQNYIHNEYTIINCMKNNSGWKISIRGNRIIWTLIDINGKTKSVF 999
FEYNIREDISEYINRWFFVTITNNLNNAKIYINGKLESNTDIKDIREVIA 1049
NGEIIFKLDGDIDRTQFIWMKYFSIFNTELSQSNIEERYKIQSYSEYLKD 1099
FWGNPLMYNKEYYMFNAGNKNSYIKLKKDSPVGEILTRSKYNQNSKYINY 1149
RDLYIGEKFIIRRKSNSQSINDDIVRKEDYIYLDFFNLNQEWRVYTYKYF 1199
KKEEMKLFLAPIYDSDEFYNTIQIKEYDEQPTYSCQLLFKKDEESTDEIG 1249
LIGIHRFYESGIVFEEYKDYFCISKWYLKEVKRKPYNLKLGCNWQFIPKD 1299
EGWTE 1304
- SEQ ID NO: 11 - BoNT/F7, amino acid sequence 
MPVNINNFNYNDPINNTTILYMKMPYYEDSNKYYKAFEIMDNVWIIPERNIIGKKPSDFYPPISLD
SGSSAYYDPNYLTTDAEKDRFLKTVIKLFNRINSNPAGQVLLEEIKNGKPYLGNDHTAVNEFCANN
RSTSVEIKESKGTTDSMLLNLVILGPGPNILECSTFPVRIFPNNIAYDPSEKGFGSIQLMSFSTEY
EYAFNDNTDLFIADPAISLAHELIHVLHGLYGAKGVTNKKVIEVDQGALMAAEKDIKIEEFITFGG
QDLNIITNSTNQKIYDNLLSNYTAIASRLSQVNINNSALNTTYYKNFFQWKYGLDQDSNGNYTVNI
SKFNAIYKKLFSFTECDLAQKFQVKNRSNYLFHFKPFRLLDLLDDNIYSISEGFNIGSLRVNNNGQ
NINLNSRIVGPIPDNGLVERFVGLCKSIVSKKGTKNSLCIKVNNRDLFFVASESSYNENGINSPKE
IDDTTITNNNYKKNLDEVILDYNSDAIPNLSSRLLNTTAQNDSYVPKYDSNGTSEIKEYTVDKLNV
FFYLYAQKAPEGESAISLTSSVNTALLDASKVYTFFSSDFINTVNKPVQAALFISWIQQVINDFTT
EATQKSTIDKIADISLVVPYVGLALNIGNEVQKGNFKEAIELLGAGILLEFVPELLIPTILVFTIK
SFINSDDSKNKIIKAINNALRERELKWKEVYSWIVSNWLTRINTQFNKRKEQMYQALQNQVDGIKK
IIEYKYNNYTLDEKNRLKAEYNIYSIKEELNKKVSLAMQNIDRFLTESSISYLMKLINEAKINKLS
EYDKRVNQYLLNYILENSSTLGTSSVQELNNLVSNTLNNSIPFELSEY
TNDKILISYFNRFYKRIIDSSILNMKYENNRFIDSSGYGSNISINGDIYIYSTNRNQFGIYSSRL
SEVNITQNNTIIYNSRYQNFSVSFWVRIPKYNNLKNLNNEYTIINCMRNNNSGWKISLNYNNIIW
TLQDTTGNNQKLVFNYTQMIDISDYINKWTFVTITNNRLGHSKLYINGNLTDQKSILNLGNIHVD
 
DNILFKIVGCNDTRYVGIRYFKIFNMELDKTEIETLYHSEPDSTILKDFWGNYLLYNKKYYLLNL
LKPNMSVTKNSDILNINRQRGIYSKTNIFSNARLYTGVEVIIRKVGSTDTSNTDNFVRKNDTVYI
NVVDGNSEYQLYADVSTSAVEKTIKLRRISNSNYNSNQMIIMDSIGDNCTMNFKTNNGNDIGLLG
FHLNNLVASSWYYKNIRNNTRNNGCFWSFISKEHGWQE
- SEQ ID NO: 12 - BoNT/F1 (H1241K), amino acid sequence. 
MPVVINSFNYNDPVNDDTILYMQIPYEEKSKKYYKAFEIMRNVWIIPERNTIGTDPSDFDPPASL
ENGSSAYYDPNYLTTDAEKDRYLKTTIKLFKRINSNPAGEVLLQEISYAKPYLGNEHTPINEFHP
VTRTTSVNIKSSTNVKSSIILNLLVLGAGPDIFENSSYPVRKLMDSGGVYDPSNDGFGSINIVTF
SPEYEYTFNDISGGYNSSTESFIADPAISLAHELIHALHGLYGARGVTYKETIKVKQAPLMIAEK
PIRLEEFLTFGGQDLNIITSAMKEKIYNNLLANYEKIATRLSRVNSAPPEYDINEYKDYFQWKYG
LDKNADGSYTVNENKFNEIYKKLYSFTEIDLANKFKVKCRNTYFIKYGFLKVPNLLDDDIYTVSE
GFNIGNLAVNNRGQNIKLNPKIIDSIPDKGLVEKIVKFCKSVIPRKGTKAPPRLCIRVNNRELFF
VASESSYNENDINTPKEIDDTTNLNNNYRNNLDEVILDYNSETIPQISNQTLNTLVQDDSYVPRY
DSNGTSEIEEHNVVDLNVFFYLHAQKVPEGETNISLTSSIDTALSEESQVYTFFSSEFINTINKP
VHAALFISWINQVIRDFTTEATQKSTFDKIADISLVVPYVGLALNIGNEVQKENFKEAFELLGAG
ILLEFVPELLIPTILVFTIKSFIGSSENKNKIIKAINNSLMERETKWKEIYSWIVSNWLTRINTQ
FNKRKEQMYQALQNQVDAIKTVIEYKYNNYTSDERNRLESEYNINNIREELNKKVSLAMENIERF
ITESSIFYLMKLINEAKVSKLREYDEGVKEYLLDYISEHRSILGNSVQELNDLVTSTLNNSIPFE
LSSYTNDKILILYFNKLYKKIKDNSILDMRYENNKFIDISGYGSNISINGDVYIYSTNRNQFGIY
SSKPSEVNIAQNNDIIYNGRYQNFSISFWVRIPKYFNKVNLNNEYTIIDCIRNNNSGWKISLNYN
KIIWTLQDTAGNNQKLVFNYTQMISISDYINKWIFVTITNNRLGNSRIYINGNLIDEKSISNLGD
IHVSDNILFKIVGCNDTRYVGIRYFKVFDTELGKTEIETLYSDEPDPSILKDFWGNYLLYNKRYY
LLNLLRTDKSITQNSNFLNINQQRGVYQKPNIFSNTRLYTGVEVIIRKNGSTDISNTDNFVRKND
LAYINVVDRDVEYRLYADISIAKPEKIIKLIRTSNSNNSLGQIIVMDSIGNNCTMNFQNNNGGNI
GLLGFKSNNLVASSWYYNNIRKNTSSNGCFWSFISKEHGWQEN
- SEQ ID NO: 13 - BoNT/F7-1, amino acid sequence 
MPVNINNFNYNDPINNTTILYMKMPYYEDSNKYYKAFEIMDNVWIIPERNIIGKKPSDFYPPISL
DSGSSAYYDPNYLTTDAEKDRFLKTVIKLFNRINSNPAGQVLLEEIKNGKPYLGNDHTAVNEFCA
NNRSTSVEIKESKGTTDSMLLNLVILGPGPNILECSTFPVRIFPNNIAYDPSEKGFGSIQLMSFS
TEYEYAFNDNTDLFIADPAISLAHELIHVLHGLYGAKGVTNKKVIEVDQGALMAAEKDIKIEEFI
TFGGQDLNIITNSTNQKIYDNLLSNYTAIASRLSQVNINNSALNTTYYKNFFQWKYGLDQDSNGN
YTVNISKFNAIYKKLFSFTECDLAQKFQVKNRSNYLFHFKPFRLLDLLDDNIYSISEGFNIGSLR
VNNNGQNINLNSRIVGPIPDNGLVERFVGLCKSVIPRKGTKAPPRLCIKVNNRDLFFVASESSYN
ENGINSPKEIDDTTITNNNYKKNLDEVILDYNSDAIPNLSSRLLNTTAQNDSYVPKYDSNGTSEI
KEYTVDKLNVFFYLYAQKAPEGESAISLTSSVNTALLDASKVYTFFSSDFINTVNKPVQAALFIS
 
WIQQVINDFTTEATQKSTIDKIADISLVVPYVGLALNIGNEVQKGNFKEAIELLGAGILLEFVPE
LLIPTILVFTIKSFINSDDSKNKIIKAINNALRERELKWKEVYSWIVSNWLTRINTQFNKRKEQM
YQALQNQVDGIKKIIEYKYNNYTLDEKNRLKAEYNIYSIKEELNKKVSLAMQNIDRFLTESSISY
LMKLINEAKINKLSEYDKRVNQYLLNYILENSSTLGTSSVQELNNLVSNTLNNSIPFELSEYTND
KILISYFNRFYKRIIDSSILNMKYENNRFIDSSGYGSNISINGDIYIYSTNRNQFGIYSSRLSEV
NITQNNTIIYNSRYQNFSVSFWVRIPKYNNLKNLNNEYTIINCMRNNNSGWKISLNYNNIIWTLQ
DTTGNNQKLVFNYTQMIDISDYINKWTFVTITNNRLGHSKLYINGNLTDQKSILNLGNIHVDDNI
LFKIVGCNDTRYVGIRYFKIFNMELDKTEIETLYHSEPDSTILKDFWGNYLLYNKKYYLLNLLKP
NMSVTKNSDILNINRQRGIYSKTNIFSNARLYTGVEVIIRKVGSTDTSNTDNFVRKNDTVYINVV
DGNSEYQLYADVSTSAVEKTIKLRRISNSNYNSNQMIIMDSIGDNCTMNFKTNNGNDIGLLGFHL
NNLVASSWYYKNIRNNTRNNGCFWSFISKEHGWQE
- SEQ ID NO: 14 - activation loop, amino acid sequence
   KSVIPRKGTKAPPRL

### EXAMPLES

The following Example(s) serve to illustrate particular embodiments of the invention, and do not limit the scope of the invention defined in the claims in any way.

### Example 1 - Study of Human bladder tissue samples

Briefly, Human bladder tissue samples were obtained from patients undergoing cystectomy for bladder cancer with no known bladder dysfunction according to their medical chart, ensuring that the collection and use of any tissue or other samples was carried out in accordance with all relevant laws, regulations and codes of practice, including having obtained informed consent of patients in writing (mentioning data privacy obligations), as well as patient medical history.

After surgical procedure, the samples were immediately transported from the operating room to the pathologist facilities where a normal piece of the bladder dome, i.e. with no macroscopic tumoral tissue, was selected for experiments. Immediately after removal, the tissue samples were stored at 4°C in Krebs-HEPES buffer (with the following mM composition: NaCl 118.0; KCI 4.7; MgSO₄ 1.2; KH2PO₄ 1.2; CaCl₂ 2.5; NaHCO₃ 4.2; glucose 11.1; HEPES 20.8; pH 7.4) containing penicillin (100 IU/ml) and streptomycin (0.1 mg/ml) for optimal conservation until use (within a maximum of 24 hours) and transported to the research facilities.

In order to prepare strips, the urothelium was carefully removed and eight sections of detrusor (4x2x2 mm) were excised from the bladder of each donor for each experiment. The remaining bladder tissue was weighed, divided into 2 cryotubes and immediately frozen in liquid nitrogen, then stored at -80°C for future analysis.

*Ex vivo* experiments were performed using a set-up placed under a laboratory hood (Captair Chem Filtair XL1346A) composed of organ chambers filled with Krebs-HEPES buffer maintained at 37°C and continuously bubbled with 95% O₂ and 5% CO₂ to maintain a pH at 7.4. The bladder strips were suspended in 5 ml organ chambers and were connected to force transducers for isometric tension recording (*Pioden controls Ltd, UK*). An initial tension of 1g was applied. Following amplification, the tension changes were digitalized via a Mac Lab TM/8 using Chart TM 5 software (*AD Instruments Ltd*)*.* After extensive washings, the strips were contracted by electrical field stimulation (EFS) via two platinum electrodes situated on either sides of the strips and connected to a stimulator (Bionic System Nozay, France).

The tension was adjusted during an equilibration period of 60 min to reach a resting tension of 1g, time during which the buffer solution was renewed every 15 min.

Following the equilibration period, the detrusor smooth muscle strips were primed by adding sequentially to the organ bath KCI (100 mM, 10 min) then carbachol (3.10⁻⁶ M, 10 min) with washing steps between each compound addition. An injection of 0.5 % of gelatin was then performed into the organ bath prior to the application of EFS trains.

EFS trains (20 Hz, 1 ms pulse duration, 5 s train duration, 300 mA) were continuously performed by groups of 3 stimulations applied at 1-min interval and followed by a 3-min period of rest (conditions of stimulation based on our experience with human bladder tissue and selected to give robust and stable contractions in human bladder strips). Stimulations were continued until stable responses were obtained (a response was considered stable when the percentage of variation of the amplitude of EFS contractions calculated for the last three groups of EFS contraction during stabilization period was ≥ 90% or ≤ 110%).

Individual strips were incubated with vehicle (Krebs with 0.5 % gelatin), 0.1, 1, 3, 5 or 10 nM of a botulinum neurotoxin and the EFS stimulations were continued for 3 hours. At the end of the experiment, bladder strips were contracted by direct activation of the muscarinic receptor by adding carbachol (3.10⁻⁶ M, 10 min) to the organ bath. The comparison of carbacholinduced strip contractions before and after botulinum neurotoxins incubation acts as a viability test of the strips during the experiment.

**Table 2 - Protocol for human bladder tissue sample studies**

| Strip 1 | Strip 2 | Strip 3 | Strip 4 | Strip 5 | Strip 6 | Strip 7 when possible | Strip 8 when possible |
|---|---|---|---|---|---|---|---|
| Equilibration 60 min | | | | | | | |
| KCI (100 mM, 10 min) | | | | | | | |
| Wash | | | | | | | |
| Carbachol (3.10⁻⁶ M, 10 min) | | | | | | | |
| Wash | | | | | | | |
| Gelatin** injection into the bath: 25µl/5ml | | | | | | | |
| Repetitive EFS (20Hz - 1ms, 5s train duration, 300 mA, 3-min interval) until stable responses are obtained | | | | | | | |
| | | | Incubation for 3 hrs with Vehicle, BoNT (1, 3, 5, 10 nM) | | | | |
| | | | Repetitive EFS (20Hz - 1ms, 5s train duration, 300 mA, 3-min interval) for 3 hrs | | | | |
| | | | Carbachol (3.10⁻⁶ M, 10 min) | | | | |

| | | | | | | | |
|---|---|---|---|---|---|---|---|
| ** Gelatin Priorex^{®} Highly purified type A. | | | | | | | |

Eight non-complexed botulinum neurotoxins were tested:
- nBoNT/A, a natural botulinum neurotoxin type A obtained from List Biological Laboratories, Inc. (SEQ ID NO: 1);
- nBoNT/B, a natural botulinum neurotoxin type B obtained from List Biological Laboratories, Inc. (SEQ ID NO: 2);
- rBoNT/B_{MY}, a recombinant botulinum neurotoxin type B comprising two mutations in the H_{C} domain, E1191M and S1199Y (SEQ ID NO: 9);
- rBoNT/AB_{MY}, a recombinant chimeric botulinum neurotoxin (LH_{N} domain from BoNT/A and H_{C} domain from BoNT/B) comprising two mutations in the H_{C} domain, E1191M and S1199Y (SEQ ID NO: 10);
- nBoNT/F, a natural botulinum neurotoxin subtype F1 obtained from Metabiologics Inc. (SEQ ID NO: 6);
- mrBoNT/F, a recombinant botulinum neurotoxin subtype F1 comprising a single-point mutation in the Hcc domain, H1241K (SEQ ID NO: 12);
- mrBoNT/F7-1, a recombinant chimeric botulinum neurotoxin subtype F7, in which the native BoNT/F7 activation loop has been replaced with the BoNT/F1 activation loop (SEQ ID NO: 13).

The incubation of botulinum neurotoxins in the organ bath was performed by adding 0.75 µl, 7.5 µl, 22.5 µl, 37.5 µl or 75 µl of the botulinum toxin stock solutions (at 666 nM) to 5 ml Krebs-HEPES buffer with 0.5% gelatin to obtain 0.1, 1, 3, 5 or 10 nM respectively as final concentration in the organ bath.

Contractile responses to pharmacological agents were quantified in terms of mean developed tension. Mean developed tension (mg, corresponding to the mean of data point in a selected tracing) was measured during a 1-minute section before and at the end of the stabilized response of each strip following either KCI or carbachol addition.

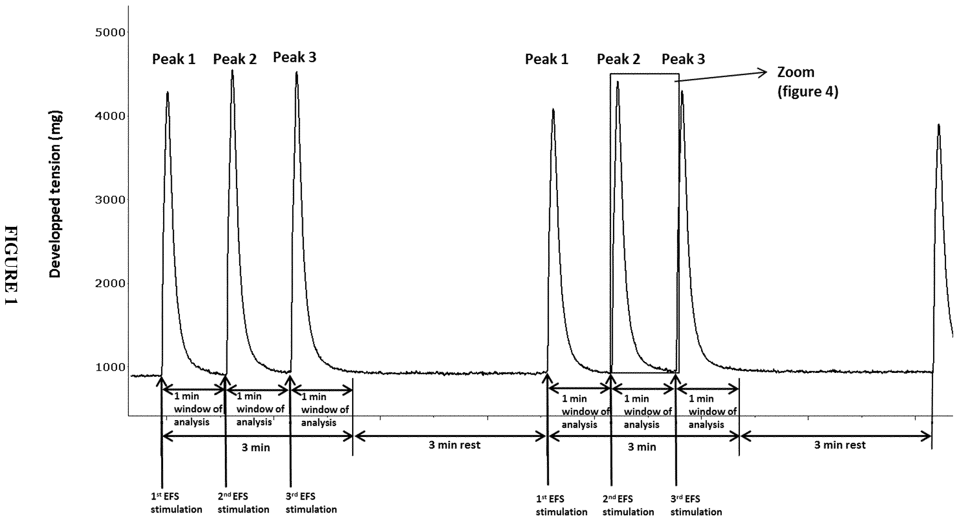

Contractile responses to EFS-induced contractions were quantified as follows. In summary, 3 stimulations were applied at 1-min interval followed by 3 min rest, as shown in figure 1.

Each EFS-induced response was analysed during the time frame between 2 consecutive stimulations i.e. 60 seconds. This analysis was performed on the last two responses of every group of stimulation for 3 hours.

For each group of stimulation, values obtained on the last two responses (peak 2 and 3) were averaged.

To quantify the EFS-induced responses, the amplitude of EFS-induced contraction (mg) was calculated as the difference between developed tension before stimulation and maximal developed tension during response to EFS, i.e. the difference between the largest (Max) and the smallest (Min) data points in the selection.

These amplitude values were expressed as a percentage of the value corresponding to the maximal EFS contraction measured during the last train of stimulation during the stabilization period before the addition of botulinum neurotoxins.

The inhibitory effects of botulinum neurotoxins were evaluated by determining the paralysis time 50 (T50) (min) values i.e., the time necessary to inhibit 50% of the maximal response. These values were computed using GraphPad Prism 6.05 variable slope model (Y=Bottom + (Top-Bottom)/(1+10^((LogIC50-X)*HillSlope)). It is also called a four-parameter doseresponse logistic curve.

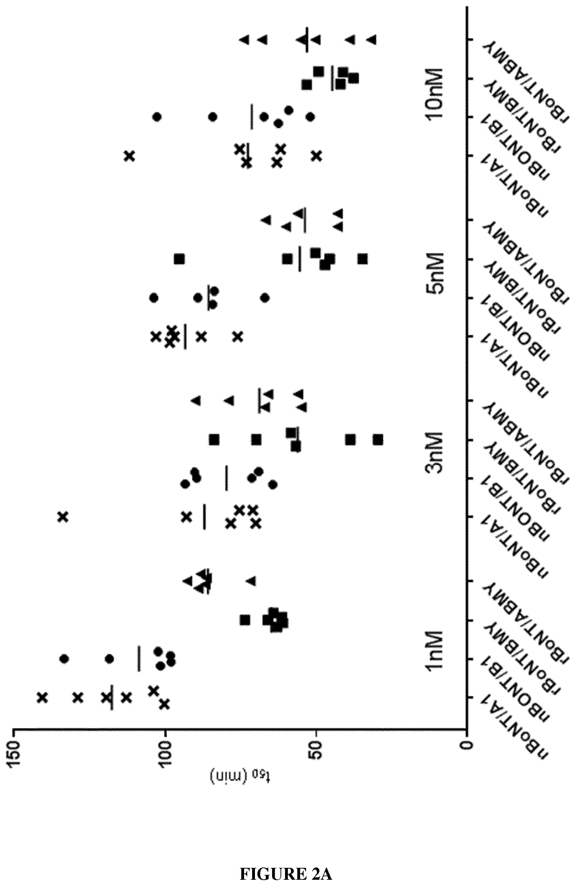

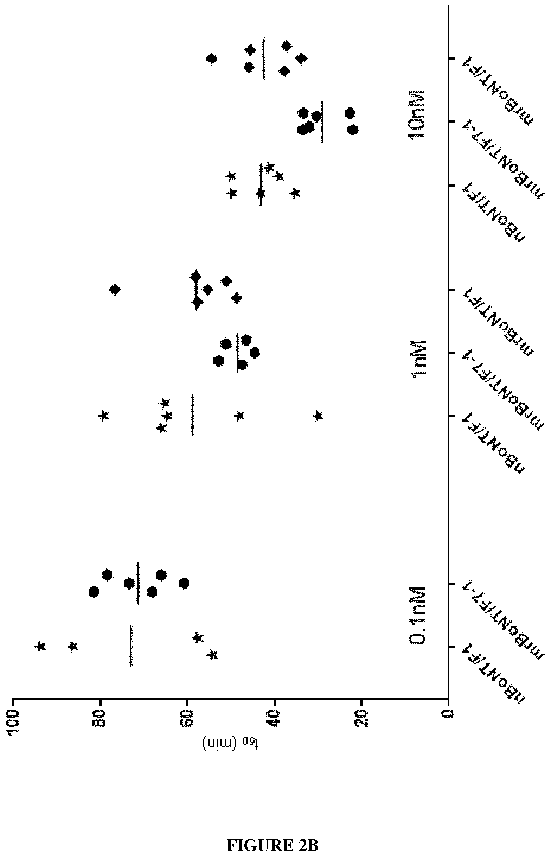

The results are presented in table 3 and in figure 2.

**Table 3 - Inhibitory effects of botulinum neurotoxins on human bladder tissue contraction evaluated by T50 (min) from Amplitude Contraction Inhibition**

| **BoNT** | **Concentration (nM)** | **T50 (min) from Amplitude Contraction Inhibition** | **N** |
|---|---|---|---|
| nBoNT/F | 0.1 | 73.0 ± 10.0 | 4 |
| mrBoNT/F7-1 | 0.1 | 71.4 ± 3.2 | 6 |
| nBoNT/A | 1 | 117.8 ± 6.3 | 6 |
| nBoNT/B | 1 | 108.8 ± 5.8 | 6 |
| rBoNT/B_{MY} | 1 | 64.9 ± 1.9* | 6 |
| rBoNT/AB_{MY} | 1 | 85.8 ± 3.0 | 6 |
| nBoNT/F | 1 | 58.9 ± 7.1*; $ | 6 |
| mrBoNT/F | 1 | 58.0 ± 4.0** ; $$ | 5 |
| mrBoNT/F7-1 | 1 | 48.5 ± 1.5*** ; $$$ | 6 |
| nBoNT/A | 3 | 87.0 ± 10. | 6 |
| nBoNT/B | 3 | 79.6 ± 5.2 | 6 |
| rBoNT/B_{MY} | 3 | 56.2 ± 8.1* | 6 |
| rBoNT/AB_{MY} | 3 | 68.9 ± 5.5 | 6 |
| nBoNT/A | 5 | 93,5 ± 4.0 | 6 |
| nBoNT/B | 5 | 85.6 ± 5.9 | 4 |
| rBoNT/B_{MY} | 5 | 55.5 ± 8.6* | 6 |
| rBoNT/AB_{MY} | 5 | 53.8 ± 4.8* | 5 |
| nBoNT/A | 10 | 72.5 ± 8.7 | 6 |
| nBoNT/B | 10 | 71.3 ± 7.7 | 6 |
| rBoNT/B_{MY} | 10 | 44.7 ± 2.9 | 5 |
| rBoNT/AB_{MY} | 10 | 53.1 ± 6.6 | 6 |
| nBoNT/F | 10 | 43.1 ± 2.4 | 6 |
| mrBoNT/F | 10 | 42.5 ± 3.1 | 6 |
| mrBoNT/F7-1 | 10 | 29.0 ± 2.2***; $$$ | 6 |

| | | | |
|---|---|---|---|
| Kruskal-Wallis test: *, **, *** = p<0.05, p<0.01, p<0.001 versus nBoNT/A. Kruskal-Wallis test: $, $$, $$$ = p<0.05, p<0.01, p<0.001 versus nBoNT/B. nBoNT/A, nBoNT/B, rBoNT/B_{MY}, rBoNT/AB_{MY}, nBoNT/F, mrBoNT/F and mrBoNT/F7-1 displayed a concentration-dependent inhibition of EFS-induced contractile response in terms of amplitude. nBoNT/A and nBoNT/B induced comparable inhibitory effects on EFS-induced contractions of human bladder strips at the same concentration. Further, at all doses, rBoNT/B_{MY}, rBoNT/AB_{MY}, nBoNT/F, mrBoNT/F and mrBoNT/F7-1 induced a higher reduction of EFS-elicited contractions of human bladder strips compared to either nBoNT/A and/or nBoNT/B. All BoNT/Fs tested as well as rBoNT/B_{MY} and rBoNT/AB_{MY} appeared to require a lower concentration to achieve the same reduction of contractions than nBoNT/A or nBoNT/B. Of all BoNTs tested, mrBoNT/F7-1 was the one inducing the greatest reduction of contractions. | | | |

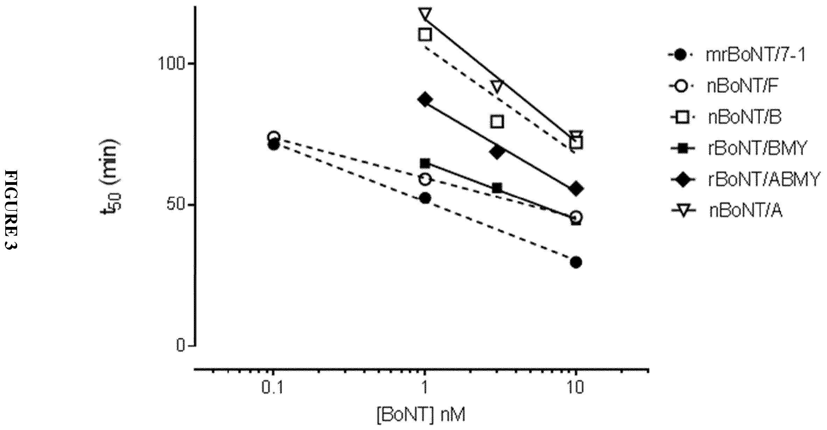

Based on the above observations, the potency of the tested botulinum toxins on human bladder tissue was quantified according to the method described by Weisemann et al. (Generation and Characterization of Six Recombinant Botulinum Neurotoxins as Reference Material to Serve in an International Proficiency Test, Toxins, 2015, 7(12): 5035-5054; doi:10.3390/toxins7124861), in particular by plotting the T50 against the botulinum toxin protein concentration, and logarithmic functions were fitted yielding excellent *R*² values, as shown in table 4 below and figure 3.

**Table 4 - Normalized potency of the botulinum neurotoxins**

| **BoNT** | **Yint** | **Slope** | **R²** | **Calculated [BoNT] (nM) giving a T50 of 70min** | **Relative potency versus nBoNT/A** | **Relative potency versus nBoNT/B** |
|---|---|---|---|---|---|---|
| nBoNT/A | 116 | -43.35 | 0.8669 | 11.511 | 1.0 | 0.8 |
| nBoNT/B | 106 | -37.82 | 0.9529 | 8.951 | 1.3 | 1.0 |
| mrBoNT/B | 65.23 | -20.23 | 0.9971 | 0.581 | 19.8 | 15.4 |
| mrBoNT/AB | 86.37 | -31.49 | 0.9836 | 3.310 | 3.5 | 2.7 |
| nBoNT/F | 59.73 | -14.15 | 0.9993 | 0.188 | 61.2 | 47.6 |
| mrBoNT/F7-1 | 51.33 | -20.9 | 0.9973 | 0.128 | 90.0 | 70.0 |

The results show that the potency of BoNT/F was the highest on human bladder tissue, followed by BoNT/B, then BoNT/A. In particular, nBoNT/B was about 1.5 times more potent than nBoNT/A; mrBoNT/B about 20 times more potent than nBoNT/A and about 15 more potent than nBoNT/B; mrBoNT/AB about 4 times more potent than nBoNT/A and about 3 times more potent than nBoNT/B; nBoNT/F about 60 times more potent than nBoNT/A and about 50 times more potent than nBoNT/B; and mrBoNT/F7-1 about 90 times more potent than nBoNT/A and about 70 times more potent than nBoNT/B. Besides, mrBoNT/F exhibited the same T50 values at 1 nM and 10 nM as nBoNT/F: it can be reasonably deduced that mrBoNT/F is as potent as nBoNT/F.

Accordingly, based on the surprising data disclosed herein, BoNT/B and BoNT/F neurotoxins, as well as clostridial neurotoxins that display similar binding, uptake, translocation and/or SNARE cleavage properties, such as BoNT/D, BoNT/D-C or BoNT/G, can be used to treat disorders of the autonomic system in humans, such as smooth muscle disorders in particular urinary disorders (NOD, OAB, etc.), at a dose lower than or equivalent to the one BoNT/A used to treat the same disorders. The relative potency values identified in table 4 can notably be relied on to determine the therapeutic quantity of BoNT/B, BoNT/F, BoNT/D, BoNT/D-C, or BoNT/G clostridial neurotoxin to administer in said subjects, based on the known doses that are capable of treating the same autonomic disorder with BoNT/A.

## Claims

1. A clostridial neurotoxin for use in the treatment of an autonomic disorder in a human patient, wherein the clostridial neurotoxin comprises an H_{CC} domain from a BoNT/B, BoNT/F, BoNT/D, BoNT/D-C, or BoNT/G, and wherein a dose ranging from 0.00025 ng to 3 ng of said clostridial neurotoxin is administered to the patient.

2. The clostridial neurotoxin for use according to claim 1, wherein said autonomic disorder is selected from smooth muscle disorders, hypersecretory disorders, respiratory disorders, inflammatory disorders with an autonomic component, neuroendocrine disorders and other autonomic disorders directly associated with a central neurological disorder.

3. The clostridial neurotoxin for use according to claim 1 or 2, wherein said H_{CC} domain is a BoNT/B H_{CC} domain or a BoNT/F H_{CC} domain.

4. The clostridial neurotoxin for use according to any one of the preceding claims, wherein said BoNT/B H_{CC} domain comprises at least one amino acid residue mutation increasing its binding affinity for the human Syt II receptor by at least 50 % as compared to the natural BoNT/B H_{CC} domain, wherein said at least one amino acid residue mutation is selected from the group consisting of 1118M, 1183M, 1191M, 1191I, 1191Q, 1191T, 1199Y, 1199F, 1199L, 1201V, 1191C, 1191V, 1191L, 1191Y, 1199W, 1199E, 1199H, 1178Y, 1178Q, 1178A, 1178S, 1183C, 1183P and any combinations thereof.

5. The clostridial neurotoxin for use according to claim 4, wherein said at least one amino acid residue mutation consists of the two amino acid mutations 1191M and 1199Y.

6. The clostridial neurotoxin for use according to any one of claims 1-3, wherein said BoNT/F H_{CC} domain comprises at least one amino acid residue mutation increasing its binding affinity for one or more gangliosides as compared to the natural BoNT/F Hcc domain, wherein said amino acid residue mutation is 1241K.

7. The clostridial neurotoxin for use according to claim 6, wherein said gangliosides are selected from GD1a and/or GM1a.

8. The clostridial neurotoxin for use according to any one of claims 1-5, wherein said clostridial neurotoxin is a BoNT/B neurotoxin.

9. The clostridial neurotoxin for use according to any one of claims 1-3, 6 or 7, wherein said clostridial neurotoxin is a BoNT/F neurotoxin.

10. The clostridial neurotoxin for use according to any one of claims 1-7, wherein said clostridial neurotoxin is a chimeric neurotoxin.

11. The clostridial neurotoxin for use according to any one of claims 1-5, wherein said clostridial neurotoxin is a chimeric neurotoxin comprising a BoNT/B H_{C} domain and a BoNT/A LH_{N} domain.

12. The clostridial neurotoxin for use according to any one of claims 1-3, 6 or 7, wherein said clostridial neurotoxin is a chimeric neurotoxin comprising an LH_{N} domain from a first BoNT/F subtype and an activation loop from a second BoNT/F subtype, and wherein said second BoNT/F subtype differs from said first BoNT/F subtype, preferably wherein said LH_{N} domain is a BoNT/F7 LH_{N} domain and/or said activation loop is a BoNT/F1 activation loop.

13. The clostridial neurotoxin for use according to any one of the preceding claims, wherein the clostridial neurotoxin comprises an amino acid sequence selected from any one of SEQ ID NOs: 2-13 or an amino acid sequence having at least 70% sequence identity thereto, preferably at least 90% or 95% sequence identity thereto.

14. The clostridial neurotoxin for use according to any one of the preceding claims, wherein the clostridial neurotoxin comprises:
a. an amino acid sequence SEQ ID NO: 13 or an amino acid sequence having at least 70% sequence identity thereto, preferably at least 90% or 95% sequence identity thereto; or
b. an amino acid sequence SEQ ID NO: 9 or an amino acid sequence having at least 70% sequence identity thereto, preferably at least 90% or 95% sequence identity thereto; or
c. an amino acid sequence SEQ ID NO: 12 or an amino acid sequence having at least 70% sequence identity thereto, preferably at least 90% or 95% sequence identity thereto; or
d. an amino acid sequence SEQ ID NO: 10 or an amino acid sequence having at least 70% sequence identity thereto, preferably at least 90% or 95% sequence identity thereto; or
e. an amino acid sequence SEQ ID NO: 6 or an amino acid sequence having at least 70% sequence identity thereto, preferably at least 90% or 95% sequence identity thereto.

## Patentansprüche

1. Ein Clostridium-Neurotoxin zur Verwendung bei der Behandlung einer autonomen Störung bei einem menschlichen Patienten, wobei das Clostridium-Neurotoxin eine H_{cc}-Domäne von einem BoNT/B, BoNT/F, BoNT/D, BoNT/D-C oder BoNT/G umfasst und wobei dem Patienten eine Dosis im Bereich von 0,00025 ng bis 3 ng des genannten Clostridium-Neurotoxins verabreicht wird.

2. Clostridium-Neurotoxin zur Verwendung nach Anspruch 1, wobei die genannte autonome Störung aus Störungen der glatten Muskulatur, Hypersekretionsstörungen, Störungen der Atemwege, entzündlichen Störungen mit einer autonomen Komponente, neuroendokrinen Störungen und anderen autonomen Störungen, die direkt mit einer zentralen neurologischen Störung assoziiert sind, ausgewählt ist.

3. Clostridium-Neurotoxin zur Verwendung nach Anspruch 1 oder 2, wobei die genannte H_{cc}-Domäne eine BoNT/B-H_{cc}-Domäne oder eine BoNT/F-H_{cc}-Domäne ist.

4. Clostridium-Neurotoxin zur Verwendung nach einem der vorhergehenden Ansprüche, wobei die genannte BoNT/B-H_{cc}-Domäne mindestens eine Aminosäurerestmutation umfasst, die ihre Bindungsaffinität für den humanen Syt-II-Rezeptor im Vergleich mit der natürlichen BoNT/B-H_{cc}-Domäne um mindestens 50% steigert, wobei die genannte mindestens eine Aminosäurerestmutation aus der Gruppe ausgewählt ist, die aus 1118M, 1183M, 1191M, 1191I, 1191Q, 1191T, 1199Y, 1199F, 1199L, 1201V, 1191C, 1191V, 1191L, 1191Y, 1199W, 1199E, 1199H, 1178Y, 1178Q, 1178A, 1178S, 1183C, 1183P und Kombinationen davon besteht.

5. Clostridium-Neurotoxin zur Verwendung nach Anspruch 4, wobei die genannte mindestens eine Aminosäurerestmutation aus den zwei Aminosäuremutationen 1191M und 1199Y besteht.

6. Clostridium-Neurotoxin zur Verwendung nach einem der Ansprüche 1-3, wobei die genannte BoNT/F-H_{cc}-Domäne mindestens eine Aminosäurerestmutation umfasst, die ihre Bindungsaffinität für ein oder mehrere Ganglioside im Vergleich zu der natürlichen BoNT/F-H_{cc}-Domäne steigert, wobei die genannte Aminosäurerestmutation 1241K ist.

7. Clostridium-Neurotoxin zur Verwendung nach Anspruch 6, wobei die genannten Ganglioside aus GD1a und/oder GM1a ausgewählt sind.

8. Clostridium-Neurotoxin zur Verwendung nach einem der Ansprüche 1-5, wobei das genannte Clostridium-Neurotoxin ein BoNT/B-Neurotoxin ist.

9. Clostridium-Neurotoxin zur Verwendung nach einem der Ansprüche 1-3, 6 oder 7, wobei das genannte Clostridium-Neurotoxin ein BoNT/F-Neurotoxin ist.

10. Clostridium-Neurotoxin zur Verwendung nach einem der Ansprüche 1-7, wobei das genannte Clostridium-Neurotoxin ein chimäres Neurotoxin ist.

11. Clostridium-Neurotoxin zur Verwendung nach einem der Ansprüche 1-5, wobei das genannte Clostridium-Neurotoxin ein chimäres Neurotoxin ist, das eine BoNT/B-H_{c}-Domäne und eine BoNT/A-LH_{N}-Domäne umfasst.

12. Clostridium-Neurotoxin zur Verwendung nach einem der Ansprüche 1-3, 6 oder 7, wobei das genannte Clostridium-Neurotoxin ein chimäres Neurotoxin ist, das eine LH_{N}-Domäne von einem ersten BoNT/F-Subtyp und eine Aktivierungsschleife von einem zweiten BoNT/F-Subtyp umfasst und wobei sich der genannte zweite BoNT/F-Subtyp von dem genannten ersten BoNT/F-Subtyp unterscheidet, wobei die genannte LH_{N}-Domäne bevorzugt eine BoNT/F7-LH_{N}-Domäne ist und/oder die genannte Aktivierungsschleife eine BoNT/F1-Aktivierungsschleife ist.

13. Clostridium-Neurotoxin zur Verwendung nach einem der vorhergehenden Ansprüche, wobei das Clostridium-Neurotoxin eine Aminosäuresequenz umfasst, die aus einer von SEQ ID NOs: 2-13 oder einer Aminosäuresequenz, die eine mindestens 70%ige Sequenzidentität damit, bevorzugt eine mindestens 90%ige oder 95%ige Sequenzidentität damit aufweist, ausgewählt ist.

14. Clostridium-Neurotoxin zur Verwendung nach einem der vorhergehenden Ansprüche, wobei das Clostridium-Neurotoxin Folgendes umfasst:
a. eine Aminosäuresequenz SEQ ID NO: 13 oder eine Aminosäuresequenz, die eine mindestens 70%ige Sequenzidentität damit, bevorzugt eine mindestens 90%ige oder 95%ige Sequenzidentität damit aufweist; oder
b. eine Aminosäuresequenz SEQ ID NO: 9 oder eine Aminosäuresequenz, die eine mindestens 70%ige Sequenzidentität damit, bevorzugt eine mindestens 90%ige oder 95%ige Sequenzidentität damit aufweist; oder
c. eine Aminosäuresequenz SEQ ID NO: 12 oder eine Aminosäuresequenz, die eine mindestens 70%ige Sequenzidentität damit, bevorzugt eine mindestens 90%ige oder 95%ige Sequenzidentität damit aufweist; oder
d. eine Aminosäuresequenz SEQ ID NO: 10 oder eine Aminosäuresequenz, die eine mindestens 70%ige Sequenzidentität damit, bevorzugt eine mindestens 90%ige oder 95%ige Sequenzidentität damit aufweist; oder
e. eine Aminosäuresequenz SEQ ID NO: 6 oder eine Aminosäuresequenz, die eine mindestens 70%ige Sequenzidentität damit, bevorzugt eine mindestens 90%ige oder 95%ige Sequenzidentität damit aufweist.

## Revendications

1. Neurotoxine clostridienne destinée à une utilisation dans le traitement d'un trouble autonomique chez un patient humain, la neurotoxine clostridienne comprenant un domaine H_{cc} d'un sérotype BoNT/B, BoNT/F, BoNT/D, BoNT/D-C ou BoNT/G, et une dose dans la plage de 0,00025 ng à 3 ng de ladite neurotoxine clostridienne étant administrée au patient.

2. Neurotoxine clostridienne destinée à une utilisation selon la revendication 1, ledit trouble autonomique étant sélectionné parmi des affections touchant les muscles lisses, des affections hypersécrétoires, des troubles respiratoires, des affections inflammatoires ayant une composante autonomique, des troubles neuroendocriniens et d'autres troubles autonomiques directement associés à une pathologie du système nerveux central.

3. Neurotoxine clostridienne destinée à une utilisation selon la revendication 1 ou 2, ledit domaine H_{cc} étant un domaine H_{cc} de BoNT/B ou un domaine H_{cc} de BoNT/F.

4. Neurotoxine clostridienne destinée à une utilisation selon l'une quelconque des revendications précédentes, ledit domaine H_{cc} de BoNT/B comprenant au moins une mutation de résidu acide aminé augmentant son affinité de liaison pour le récepteur humain Syt II d'au moins 50 % par rapport au domaine H_{cc} naturel de BoNT/B, l'au moins une mutation de résidu acide aminé étant sélectionnée dans le groupe constitué de 1118M, 1183M, 1191M, 1191I, 1191Q, 1191T, 1199Y, 1199F, 1199L, 1201V, 1191C, 1191V, 1191L, 1191Y, 1199W, 1199E, 1199H, 1178Y, 1178Q, 1178A, 1178S, 1183C, 1183P et de n'importe quelles combinaisons de celles-ci.

5. Neurotoxine clostridienne destinée à une utilisation selon la revendication 4, ladite au moins une mutation de résidu acide aminé consistant en les deux mutations d'acides aminés 1191M et 1199Y.

6. Neurotoxine clostridienne destinée à une utilisation selon l'une quelconque des revendications 1 à 3, ledit domaine H_{cc} de BoNT/F comprenant au moins une mutation de résidu acide aminé augmentant son affinité de liaison pour un ou plusieurs gangliosides par rapport au domaine H_{cc} naturel de BoNT/F, ladite mutation de résidu acide aminé étant 1241K.

7. Neurotoxine clostridienne destinée à une utilisation selon la revendication 6, lesdits gangliosides étant sélectionnés pami GD1a et/ou GM1a.

8. Neurotoxine clostridienne destinée à une utilisation selon l'une quelconque des revendications 1 à 5, ladite neurotoxine clostridienne étant une neurotoxine BoNT/B.

9. Neurotoxine clostridienne destinée à une utilisation selon l'une quelconque des revendications 1 à 3, 6 ou 7, ladite neurotoxine clostridienne étant une neurotoxine BoNT/F.

10. Neurotoxine clostridienne destinée à une utilisation selon l'une quelconque des revendications 1 à 7, ladite neurotoxine clostridienne étant une neurotoxine chimérique.

11. Neurotoxine clostridienne destinée à une utilisation selon l'une quelconque des revendications 1 à 5, ladite neurotoxine clostridienne étant une neurotoxine chimérique comprenant un domaine H_{c} de BoNT/B et un domaine LH_{N} de BoNT/A.

12. Neurotoxine clostridienne destinée à une utilisation selon l'une quelconque des revendications 1 à 3, 6 ou 7, ladite neurotoxine clostridienne étant une neurotoxine chimérique comprenant un domaine LH_{N} d'un premier sous-type de BoNT/F et une boucle d'activation d'un deuxième sous-type de BoNT/F, et ledit deuxième sous-type de BoNT/F étant différent dudit premier sous-type de BoNT/F, de préférence, ledit domaine LH_{N} étant un domaine LH_{N} de BoNT/F7 et/ou ladite boucle d'activation étant une boucle d'activation de BoNT/F1.

13. Neurotoxine clostridienne destinée à une utilisation selon l'une quelconque des revendications précédentes, la neurotoxine clostridienne comprenant une séquence d'acides aminés sélectionnée parmi l'une quelconque des SÉQ. ID n^{os} 2 à 13 ou une séquence d'acides aminés ayant une identité de séquence d'au moins 70 % avec celle-ci, de préférence ayant une identité de séquence d'au moins 90 % ou 95 % avec celle-ci.

14. Neurotoxine clostridienne destinée à une utilisation selon l'une quelconque des revendications précédentes, la neurotoxine clostridienne comprenant :
a. une séquence d'acides aminés SÉQ. ID n° 13 ou une séquence d'acides aminés ayant une identité de séquence d'au moins 70 % avec celle-ci, de préférence ayant une identité de séquence d'au moins 90 % ou 95 % avec celle-ci ; ou
b. une séquence d'acides aminés SÉQ. ID n° 9 ou une séquence d'acides aminés ayant une identité de séquence d'au moins 70 % avec celle-ci, de préférence ayant une identité de séquence d'au moins 90 % ou 95 % avec celle-ci ; ou
c. une séquence d'acides aminés SÉQ. ID n° 12 ou une séquence d'acides aminés ayant une identité de séquence d'au moins 70 % avec celle-ci, de préférence ayant une identité de séquence d'au moins 90 % ou 95 % avec celle-ci ; ou
d. une séquence d'acides aminés SÉQ. ID n° 10 ou une séquence d'acides aminés ayant une identité de séquence d'au moins 70 % avec celle-ci, de préférence ayant une identité de séquence d'au moins 90 % ou 95 % avec celle-ci ; ou
e. une séquence d'acides aminés SÉQ. ID n° 6 ou une séquence d'acides aminés ayant une identité de séquence d'au moins 70 % avec celle-ci, de préférence ayant une identité de séquence d'au moins 90 % ou 95 % avec celle-ci.
